# EUROPEAN PATENT APPLICATION

(11) **EP 4 721 693 A1**
(43) Date of publication of application: **08.04.2026**
(21) Application number: 25205220.4
(22) Date of filing: 29.09.2025
(51) Int. Cl.: A61B 34/30, A61B 34/10, A61B 34/32, A61B 34/00, A61B 90/00, B25J 9/16, B25J 19/00, G06N 3/02, G06N 20/00, A61B 17/00, A61B 34/20, A61B 34/37

(54) **ROBOTIC SURGICAL SYSTEMS EMPLOYING MACHINE LEARNING MODELS TO CHARACTERIZE TOOL INTERACTIONS**

(30) Priority: 30.09.2024 US 202463700968 P; 27.01.2025 US 202563749842 P
(71) Applicant: MAKO Surgical Corp., Weston, FL 33331 (US)
(72) Inventor: Talasaz, Ali, Plantation, 33325-2514 (US)
(74) Representative: Schott, Jakob Valentin

(57) **Abstract**

Surgical systems and methods involving machine learning model(s) to characterize tool interactions. The tool may be coupled to a robotic manipulator and configured to manipulate a tissue. A sensing system measures displacements of the tool, velocities of the tool, and interaction forces applied to the tool. A control system controls the manipulator to move the tool to interact with the tissue. Responsive to interactions of the tool with the tissue over time, the control system obtains measured values of the displacements, velocities, and interaction forces, from the sensing system. The control system implements the machine learning model to receive and process the measured values to estimate stiffness/damping parameters of the tissue and evaluate changes in the estimated stiffness/damping parameters to generate a characterization about the tissue or the tool's interaction with the tissue. The control system controls the manipulator and/or the tool based on the characterization.

## Description

### CROSS-REFERENCE TO RELATED APPLICATIONS

The subject application claims priority to and all the benefits of United States Provisional Patent Application No. 63/700,968, filed September 30, 2024, and United States Provisional Patent Application No. 63/749,842 filed January 27, 2025, the entire contents of each of the aforementioned applications being hereby incorporated by reference.

### BACKGROUND

Surgical robotic systems typically include a robotic arm that supports and moves a surgical tool to assist with performing a surgical procedure on a surgical site. Such surgical tools are often utilized to manipulate tissue. In robotic joint arthroplasty, for example, the robotic arm controls the surgical tool to sculpt or resect bone in preparation for receiving an implant. The surgical site is a crowded workspace and limited to the incision size, thereby making the site difficult to robotically navigate. The target bone may be surrounded by cartilage, soft tissue (e.g., ligaments, tendons, fatty tissue, blood vessels, etc.), tissue retractors, and the like.

Despite the intricacy of objects/tissues at the surgical site, conventional surgical robots are ill-equipped to understand the true nature of the objects/tissues with which the tool interacts. One primary reason for such limitation is that conventional surgical robots fail to recognize, much less account for, the unknown contact dynamics stemming from the tool-tissue interaction. Such unknown contact dynamics can result from complex factors, such as the properties of the tissue, friction, contact forces, or geometry variations. For example, prior surgical robots fail to characterize the nature of the tissue with which the tool interacts or distinguish different tissue types. Prior surgical robotic control systems are often non-dynamic and non-adaptable, instead relying on predetermined control models for a "one size fits all" approach to controlling the tool relative to the tissue. Such conventional surgical robotic control systems typically include simple feedback control responsive to tool position or tool load relative to the tissue. Such approaches are simply based on tool measurements and fail to make any intelligent predictions about the contact dynamics occurring between the tool and the tissue.

Accuracy of the surgical tool, particularly relative to the surgical site, is immensely important. Yet, by failing to characterize the unknown tool-tissue contact dynamics, prior surgical robots are susceptible to tool inaccuracies. Inaccuracies of only a few millimeters can cause sub-optimal control or performance of the surgical robot and/or the surgical tool. As a result, such inaccuracies may cause disturbances to the surgeon, or worse, complications during surgery or a sub-optimal surgical outcome for the patient. Furthermore, by not addressing unknown tool-tissue contact dynamics, prior surgical robots may cause the surgical tool to mistakenly contact a prohibited tissue region or tissue type. Moreover, this limitation inhibits the ability of prior surgical robots to perform higher order tasks of greater complexity.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical manipulator is provided that controls a surgical tool to interact with tissue. A control system obtains sensed measurements during interactions of the surgical tool with the tissue and applies the sensed measurements to a machine learning model that is configured to characterize a feature or a parameter of the tissue.

According to a second aspect, a surgical manipulator is provided that controls a surgical tool to interact with tissue. A control system obtains sensed measurements during interactions of the surgical tool with the tissue and applies the sensed measurements to a machine learning model that is configured to characterize a type of the tissue.

According to a third aspect, a surgical manipulator is provided that controls a surgical tool to interact with tissue. A control system obtains sensed measurements during interactions of the surgical tool with the tissue and applies the sensed measurements to a machine learning model that is configured to distinguish different tissue types.

According to a fourth aspect, a surgical manipulator is provided that controls a surgical tool to interact with tissue. A control system obtains sensed measurements during interactions of the surgical tool with the tissue and applies the sensed measurements to a machine learning model that is configured to characterize a parameter of the tissue, wherein the parameter includes one or more of: geometry, size, shape, depth, and thickness.

According to a fifth aspect, a surgical manipulator is provided that controls a surgical tool to interact with tissue. A control system obtains sensed measurements during interactions of the surgical tool with the tissue and applies the sensed measurements to a machine learning model that is configured to characterize a parameter of the tissue, wherein the parameter includes one or more of: density, stiffness, hardness, and softness.

According to a sixth aspect, a surgical manipulator is provided that controls a surgical tool to interact with tissue. A control system obtains sensed measurements during interactions of the surgical tool with the tissue and applies the sensed measurements to a machine learning model that is configured to characterize one or more of: an environment of the tissue, a layering of the tissue, and identification of the tissue being an embedded object.

According to a seventh aspect, a surgical manipulator is provided that controls a surgical tool to interact with tissue. A control system obtains sensed measurements during interactions of the surgical tool with the tissue and applies the sensed measurements to a machine learning model that is configured to characterize an interaction type of the surgical tool, wherein the interaction type comprises a soft tissue interaction, a bone tissue interaction, or a non-contact interaction.

According to an eighth aspect, a surgical system is provided, comprising: a robotic manipulator comprising a plurality of links and joints; a surgical tool coupled to the robotic manipulator and being configured to manipulate a tissue of a patient; a sensing system configured to measure: displacements of the surgical tool, velocities of the surgical tool, and interaction forces applied to the surgical tool; and a control system coupled to the robotic manipulator and the sensing system and being configured to: control the robotic manipulator to move the surgical tool to interact with the tissue; responsive to interactions of the surgical tool with the tissue, obtain, from the sensing system, measured values of the displacements, velocities, and interaction forces; input the measured values into a machine learning model that is configured to estimate stiffness and/or damping parameters of the tissue.

According to a ninth aspect, a surgical system is provided, comprising: a robotic manipulator comprising a plurality of links and joints; a surgical tool coupled to the robotic manipulator and being configured to manipulate a tissue of a patient; a sensing system configured to measure: displacements of the surgical tool, velocities of the surgical tool, and interaction forces applied to the surgical tool; and a control system coupled to the robotic manipulator and the sensing system and being configured to: control the robotic manipulator to move the surgical tool to interact with the tissue; responsive to interactions of the surgical tool with the tissue, obtain, from the sensing system, measured values of the displacements, velocities, and interaction forces; input the measured values into a machine learning model that is configured to predict an interaction characterization; and control the robotic manipulator and/or the surgical tool based on the interaction characterization.

According to a tenth aspect, a surgical system is provided, comprising: a robotic manipulator comprising a plurality of links and joints; a surgical tool coupled to the robotic manipulator and being configured to manipulate a tissue of a patient; a sensing system configured to measure: displacements of the surgical tool, velocities of the surgical tool, and interaction forces applied to the surgical tool; and a control system coupled to the robotic manipulator and the sensing system and being configured to: control the robotic manipulator to move the surgical tool to interact with the tissue; responsive to interactions of the surgical tool with the tissue over time, obtain, from the sensing system, measured values of the displacements, velocities, and interaction forces; and implement a machine learning model that is configured to: receive and process the measured values to estimate stiffness and damping parameters of the tissue; and monitor changes in the estimated stiffness and damping parameters to predict a tissue characterization; and wherein the control system is configured to control the robotic manipulator and/or the surgical tool based on the tissue characterization.

According to an eleventh aspect, a surgical manipulator is provided that controls a surgical tool to interact with tissue. A control system obtains sensed measurements during interactions of the surgical tool with the tissue and applies the sensed measurements to a machine learning model that is configured to identify a foreign body object located on, or embedded within, the tissue. The foreign body object can include one or more of: an existing implant, a piece of metal, a surgical fastener, and another surgical tool.

According to a twelfth aspect, a surgical system is provided, comprising: a robotic manipulator comprising a plurality of links and joints; a surgical tool coupled to the robotic manipulator and being configured to manipulate a tissue of a patient; a sensing system configured to measure: displacements of the surgical tool, velocities of the surgical tool, and interaction forces applied to the surgical tool; and a control system coupled to the robotic manipulator and the sensing system and being configured to: control the robotic manipulator to move the surgical tool to interact with the tissue; responsive to interactions of the surgical tool with the tissue, obtain, from the sensing system, measured values of the displacements, velocities, and interaction forces; input the measured values into a machine learning model that is configured to predict a characterization of a foreign body object located on, or embedded within, the tissue; and control the robotic manipulator and/or the surgical tool based on the characterization of the foreign body object. The foreign body object can include one or more of: an existing implant, a piece of metal, a surgical fastener, and another surgical tool.

Also provided are: a computer-implemented method for performing any of the steps implemented by the surgical system or the control system of any one or more of the preceding aspects; a non-transitory computer readable medium or computer program product, comprising instructions, which when executed by one or more processors, are configured to implement the surgical system or the control system of any one or more of the preceding aspects; the control system of any one or more of the preceding aspects; and the surgical system of any one or more of the preceding aspect further including a navigation system to track the tissue.

Any of the aspects described above can be combined in part, or in whole. Any of the above aspects can be utilized in part, or in whole, with any of the following implementations:
The machine learning model can be configured to estimate stiffness parameters of the tissue (without damping), or vice versa. The machine learning model can include a first neural network. The first neural network can include an input layer, hidden layers, and an output layer. The input layer of the first neural network can receive the measured values. The output layer of the first neural network can output the estimated stiffness and damping parameters. Optionally, the machine learning model can include or be coupled to a first long short-term memory (LSTM) or recurrent neural network (RNN). The first LSTM/RNN can be coupled to the input layer of the first neural network. The first LSTM/RNN can receive the measured values over time. The first LSTM/RNN can modify weights and biases to learn long-term dependencies among the measured values and to selectively filter the measured values. The first LSTM/RNN can provide filtered measured values to the input layer of the first neural network. The estimated stiffness and damping parameters can be embeddings of lower dimensionality than the measured values.

The machine learning model can include a second neural network. The second neural network can include an input layer, hidden layers, and an output layer. The input layer of the second neural network can receive the estimated stiffness and damping parameters. The output layer of the second neural network can output the tissue characterization. The machine learning model can optionally include or be coupled to a second LSTM/RNN. The second LSTM/RNN can be coupled to the input layer of the second neural network. The second LSTM/RNN can receive, over time, the estimated stiffness, and damping parameters from the output layer of the first neural network. The second LSTM/RNN can modify weights and biases to learn long-term dependencies among the estimated stiffness and damping parameters and to selectively filter the estimated stiffness and damping parameters. The second LSTM/RNN can provide filtered estimated stiffness and damping parameters to the input layer of the second neural network. The second LSTM/RNN can further receive, over time, measured values of the velocities of the surgical tool. The tissue characterization can be represented by a vector and can be of higher dimensionality than the estimated stiffness and damping parameters.

The tissue characterization can include a parameter of the tissue. The parameter can include geometrical features, material properties, material types, etc. The parameter of the tissue can include one or more of: geometry, size, shape, depth, and thickness. The parameter of the tissue can include one or more of: density, stiffness, hardness, softness, smoothness, roughness. The tissue characterization can include one or more of: an environment of the tissue, a layering of the tissue, and identification of the tissue being an embedded object.

The tissue characterization can include a tissue type. The tissue type can include one or more of: bone, an osteophyte, cartilage, soft tissue, muscle, ligament tissue, tendon tissue, a blood vessel, healthy tissue, and malignant tissue. The machine learning model can monitor changes in the estimated stiffness and damping parameters to predict a first tissue type and to predict a second tissue type. The first tissue type can be any one of the described tissue types and the second tissue type can any other one of the described tissue types. For example, the first tissue type can be bone, and the second tissue type can be soft tissue. The first tissue type can be bone, and the second tissue type can be cartilage. The first tissue type can be healthy tissue, and the second tissue type can be malignant tissue.

The control system can record positions of the surgical tool responsive to interactions of the surgical tool with the tissue over time. The control system can utilize the recorded positions to register the tissue characterization to locations on the tissue.

The control system can record positions of the surgical tool responsive to interactions of the surgical tool with the first tissue type and the second tissue type over time. The control system can utilize the recorded positions to register the first tissue type and the second tissue type to locations on the tissue. The control system can generate a virtual boundary to delineate a first region of the tissue comprising the first tissue type from a second region of the tissue comprising the second tissue type. The control system can register the virtual boundary to the tissue. The control system can utilize the virtual boundary to constrain movement or operation of the robotic manipulator and/or the surgical tool relative to the virtual boundary.

The machine learning model can monitor changes in the estimated stiffness and damping parameters to predict that the tissue type is a bone. The control system can record positions of the surgical tool responsive to interactions of the surgical tool with the bone over time. The control system can utilize the recorded positions to generate a 3-D surface model of the bone. The control system can register the 3-D surface model to the bone.

The control system can control the robotic manipulator and/or the surgical tool based on the tissue characterization by being configured to perform one or more of the following: modify a tool path of the surgical tool; modify a feed rate of the surgical tool; modify a cutting speed of the surgical tool; adjust a commanded pose of the surgical tool; halt movement of the surgical tool; move the surgical tool towards or away from the tissue; and/or generate or modify a virtual haptic setting for the surgical tool.

The measured displacements of the surgical tool can be indicative of measured displacements of the surgical tool by the surgical tool. The measured velocities of the surgical tool can be indicative of velocities of the tool during displacements of the surgical tool by the surgical tool. The control system can compute a deformation of the tissue based on the measured displacements of the surgical tool and the measured interaction forces of the surgical tool. The machine learning model can receive and process values of the deformation to estimate stiffness and damping parameters of the tissue and can use deformation values instead of displacement values. The measured interaction forces can be measured directly or indirectly at the surgical tool. The sensing system can include at least one force/torque sensor to measure interaction forces applied to the surgical tool. The at least one force/torque sensor can be coupled to one or both of: a distal link of the robotic manipulator, and the surgical tool.

The manipulator can include a robotic arm with motorized joints. The manipulator can include a robotic arm with passive joints that are manually adjustable. The surgical tool is configured to manipulate the tissue. The surgical tool can be a saw, burr, reamer, router, drill, impactor, pin driver, screwdriver, tool guide, tool holder, knife, guide tube, or the like. The interaction forces are applied to the surgical tool based on interactions of the surgical tool with the tissue during cutting of the tissue.

Any of the implementations described can be combined in part, or in whole.

### DESCRIPTION OF THE DRAWINGS

Advantages of the present disclosure will be readily appreciated as the same becomes better understood by reference to the following detailed description when considered in connection with the accompanying drawings.
Figure 1 is a perspective view of a robotic surgical system, according to one implementation.
Figure 2 is a block diagram of an example control system for controlling the robotic surgical system.
Figure 3 is a functional block diagram of modules implemented by the control system, according to one implementation.
Figure 4 illustrates an example output of a boundary generator.
Figure 5 illustrates an example output of a path generator.
Figure 6 is a block diagram of an example control scheme for the manipulator, according to one implementation.
Figure 7 is a block diagram of the control system illustrating a machine learning model to characterize tool interactions, according to one implementation.
Figure 8A is an example tissue model with an embedded object and a spring-damper model of the tissue and embedded object pursuant to an applied interaction force.
Figure 8B is a set of equations that can be employed by the machine learning model to characterize tool interactions, according to one example.
Figure 9 is a block diagram of the machine learning model to characterize tool interactions, including example characterizations, according to one implementation.
Figure 10 is a block diagram of an interaction evaluator portion of the machine learning model, wherein the interaction evaluator is implemented using a neural network, according to one example.
Figure 11 is a chart illustrating example object type characterizations that can be predicted by the machine learning model, according to one example.
Figure 12 is a simplified diagram of the robotic manipulator utilizing a surgical tool to interact with a multi-layer tissue including an embedded object, according to one example.
Figures 13A-13F are charts illustrating various measurements that can be obtained by a sensing system pursuant to interaction with the multi-layer tissue example of FIG. 12.

### DETAILED DESCRIPTION

### I. Example System Overview

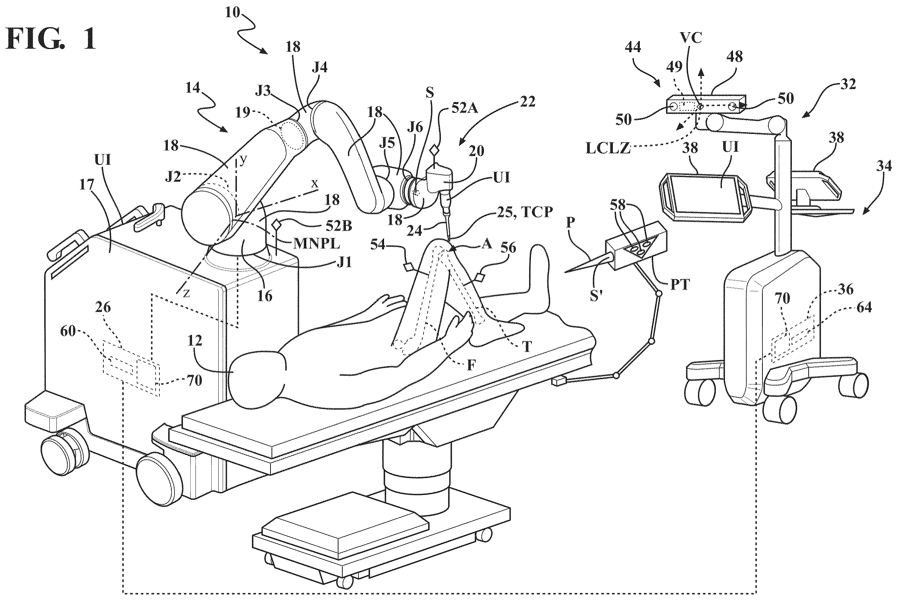

Referring to Figure 1, a surgical system 10 is illustrated. The surgical system 10 is useful for treating a surgical site or anatomical volume (A) of a patient 12, such as treating bone or soft tissue. In Figure 1, the patient 12 is undergoing a surgical procedure. The anatomy in Figure 1 includes a femur F and a tibia T of the patient 12. The surgical procedure may involve tissue removal or other forms of treatment. Treatment may include cutting, coagulating, lesioning the tissue, other in-situ tissue treatments, or the like. In some examples, the surgical procedure involves partial or total knee or hip replacement surgery, shoulder replacement surgery, spine surgery, or ankle surgery. In some examples, the surgical system 10 is designed to cut away material to be replaced by surgical implants, such as hip and knee implants, including unicompartmental, bicompartmental, multi compartmental, or total knee implants. Some of these types of implants are shown in U.S. Patent Application Publication No. 2012/0330429, entitled, "Prosthetic Implant and Method of Implantation," the disclosure of which is hereby incorporated by reference. The surgical system 10 and techniques disclosed herein may be utilized to perform other procedures, surgical or non-surgical, or may be utilized in industrial applications or other applications where robotic systems are utilized.

The surgical system 10 includes a manipulator 14 or robotic manipulator. The manipulator 14 has a base 16 and plurality of links 18. A manipulator cart 17 supports the manipulator 14 such that the manipulator 14 is fixed to the manipulator cart 17. The links 18 collectively form one or more arms of the manipulator 14. The manipulator 14 may have a serial arm configuration (as shown in Figure 1), a parallel arm configuration, or any other suitable manipulator configuration. In other examples, more than one manipulator 14 may be utilized in a multiple arm configuration. In other configurations, the manipulator 14 can include a partially or entirely adjustable arm (AA), with passive joints that are manually adjustable. The joints of the adjustable arm (AA) optionally may be locked in position by the user. In other instances, some or all the joints of the adjustable arm (AA) may be actively driven using joint motors.

The manipulator 14 can include a passive (manually articulated) joint, such as planarly extending joint. For instance, the passive joint may be the distal most joint attached to the robotic arm comprising a plurality of active joints. The planarly extending joint can support a tool such as a saw blade to allow the user to manually move the saw blade along a plane. In this case, the tool is mechanically constrained to the plane by the mechanical constraints of the passive joint, while the pose of the plane is actively constrained by the active joints of the robotic arm. In other instances, one or more joints of the manipulator 14 could be controlled to constrain the surgical tool in certain degrees of freedom, while allowing the surgical tool free passive motion in other degrees of freedom or manners, such as a rotation about a point or a selected linear motion.

The manipulator 14 can exhibit kinematic redundancy wherein the tool positions are defined by up to 6DOF, but the robotic arm may operate in more than 6DOF. With such redundancy, the manipulator 14 can utilize null-space controls whereby the joints of the manipulator 14 can change positions while maintaining the pose of the tool 20. Null-space controls can also be used to avoid singularities.

In the example shown in Figure 1, the manipulator 14 comprises a plurality of joints J and a plurality of joint encoders 19 located at the joints J for determining position data of the joints J. For simplicity, only one joint encoder 19 is illustrated in Figure 1, although other joint encoders 19 may be similarly illustrated. The manipulator 14, according to one example, has six joints J1-J6 implementing at least six-degrees of freedom (DOF) for the manipulator 14. However, the manipulator 14 may have any number of degrees of freedom and may have any suitable number of joints J and may have redundant joints.

The manipulator 14 need not require joint encoders 19 but may alternatively, or additionally, utilize motor encoders present on motors at each joint J. Also, the manipulator 14 need not require rotary joints, but may alternatively, or additionally, utilize one or more prismatic joints. Any suitable combination of joint types is contemplated.

The base 16 of the manipulator 14 is a portion of the manipulator 14 that provides a fixed reference coordinate system for other components of the manipulator 14 or the surgical system 10 in general. The origin of a manipulator coordinate system MNPL is defined at the fixed reference of the base 16. The base 16 may be defined with respect to any suitable portion of the manipulator 14, such as one or more of the links 18. Alternatively, or additionally, the base 16 may be defined with respect to the manipulator cart 17, such as where the manipulator 14 is physically attached to the manipulator cart 17. In one example, the base 16 is defined at an intersection of the axes of joints J1 and J2. Thus, although joints J1 and J2 are moving components in reality, the intersection of the axes of joints J1 and J2 is nevertheless a virtual fixed reference pose, which provides both a fixed position and orientation reference and which does not move relative to the manipulator 14 and/or manipulator cart 17.

In other examples, the manipulator 14 can be a hand-held manipulator where the base 16 is a base portion of a tool (e.g., a portion held free-hand by the user) and the tool tip is movable relative to the base portion. The base portion has a reference coordinate system that is tracked, and the tool tip has a tool tip coordinate system that is computed relative to the reference coordinate system (e.g., via motor and/or joint encoders and forward kinematic calculations). Movement of the tool tip can be controlled to follow the path since its pose relative to the path can be determined. The hand-held manipulator 14 can be like that described and shown in US20230255701, entitled "Systems and Methods for Guiding Movement of a Handheld Medical Robotic Instrument", the entire disclosure of which is hereby incorporated by reference.

The manipulator 14 and/or manipulator cart 17 house a manipulator controller 26, or other type of control unit. The manipulator controller 26 may comprise one or more computers, or any other suitable form of controller that directs the motion of the manipulator 14. The manipulator controller 26 may have a central processing unit (CPU), graphics processing unit (GPU) and/or other processors, memory, and storage. The manipulator controller 26 is loaded with software as described below. The processors could include one or more processors to control operation of the manipulator 14. The processors can be any type of microprocessor, multi-processor, and/or multi-core processing system. The manipulator controller 26 may additionally, or alternatively, comprise one or more microcontrollers, field programmable gate arrays, systems on a chip, discrete circuitry, and/or other suitable hardware, software, or firmware that is capable of carrying out the functions described herein. The term processor is not intended to limit any embodiment to a single processor. The manipulator 14 may also comprise a user interface UI with one or more displays and/or input devices (e.g., push buttons, keyboard, mouse, microphone (voice-activation), gesture control devices, touchscreens, etc.).

A tool 20 couples to the manipulator 14 and is movable relative to the base 16 to interact with the anatomy in certain modes. The tool 20 is a physical and surgical tool and is, or forms part of, an end effector 22 supported by the manipulator 14 in certain implementations. The tool 20 may be grasped by the user. One possible arrangement of the manipulator 14 and the tool 20 is described in U.S. Patent No. 9,119,655, entitled, "Surgical Manipulator Capable of Controlling a Surgical Instrument in Multiple Modes," the disclosure of which is hereby incorporated by reference. The manipulator 14 and the tool 20 may be arranged in alternative configurations. The tool 20 can be like that shown in U.S. Patent Application Publication No. 2014/0276949, filed on March 15, 2014, entitled, "End Effector of a Surgical Robotic Manipulator," hereby incorporated by reference.

The tool 20 can include an energy applicator 24 designed to contact and remove the tissue of the patient 12 at the surgical site. In one example, the energy applicator 24 is a bur 25. The bur 25 may be substantially spherical and comprise a spherical center, radius (r) and diameter. Alternatively, the energy applicator 24 may be a drill bit, a saw blade, an ultrasonic vibrating tip, or the like. The tool 20 and/or energy applicator 24 may comprise any geometric feature, e.g., perimeter, circumference, radius, diameter, width, length, volume, area, surface/plane, range of motion envelope (along any one or more axes), etc. The geometric feature may be considered to determine how to locate the tool 20 relative to the tissue at the surgical site to perform the desired treatment. In some of the embodiments described herein, a spherical bur having a tool center point (TCP) will be described for convenience and ease of illustration but is not intended to limit the tool 20 to any particular form. In other examples, the tool 20 does not include an energy applicator 24. For example, the tool 20 can be a slotted cut guide for a saw, a guide tube for receiving another tool, or the like.

The tool 20 may comprise a tool controller to control operation of the tool 20, such as to control power to the tool (e.g., to a rotary motor of the tool 20), control movement of the tool 20, control irrigation/aspiration of the tool 20, and/or the like. The tool controller may be in communication with the manipulator controller 26 or other components. The tool 20 may also comprise a user interface UI with one or more displays and/or input devices (e.g., push buttons, keyboard, mouse, microphone (voice-activation), gesture control devices, touchscreens, etc.). For example, one of the user input devices on the user interface UI of the tool 20 may be a tool input (e.g., switch or other form of user input device) that has first and second input states (see FIG. 1). The tool input can be actuated (e.g., pressed and held) by the user to be placed in the first input state and can be released to be placed in the second input state. The tool 20 may have a grip on which the tool input is located. In some versions, the tool input is a presence detector that detects the presence of a hand of the user, such as a momentary contact switch that switches between on/off states, a capacitive sensor, an optical sensor, or the like. The tool input is thus configured such that the first input state indicates that a user is actively engaging the tool 20 and the second input state indicates that the user has released the tool 20. The tool input may be a continuous activation device, i.e., inputs that must be continually actuated to allow motion of the tool 20 in the manual mode or the semi-autonomous mode, depending on which user input is actuated. For example, while the user is continually actuating the tool input, and the manual mode is enabled, the manipulator 14 will move in response to the input forces and torques applied by the user and the control system 60 will enforce the virtual boundary VB to protect the patient anatomy. When the tool input is released, input from the force/torque sensor S may be disabled such that the manipulator 14 no longer responds to the forces and torques applied by the user to the tool 20.

The manipulator controller 26 controls a state (position and/or orientation) of the tool 20 (e.g., the TCP) with respect to a coordinate system, such as the manipulator coordinate system MNPL. The manipulator controller 26 can control (linear or angular) velocity, acceleration, or other derivatives of motion of the tool 20. The tool center point (TCP), in one example, is a predetermined reference point defined at the energy applicator 24. The TCP has a known, or able to be calculated (i.e., not necessarily static), pose relative to other coordinate systems. The geometry of the energy applicator 24 is known in or defined relative to a TCP coordinate system. The TCP may be located at the spherical center of the bur 25 of the tool 20 such that only one point is tracked. The TCP may be defined in several ways depending on the configuration of the energy applicator 24. The manipulator 14 could employ the joint/motor encoders, or any other non-encoder position sensing method, to enable a pose of the TCP to be determined. The manipulator 14 may use joint measurements to determine TCP pose and/or could employ techniques to measure TCP pose directly. The control of the tool 20 is not limited to a center point. For example, any suitable primitives, meshes, etc., can be utilized to represent the tool 20.

The surgical system 10 further includes a navigation system 32. One example of the navigation system 32 is described in U.S. Patent No. 9,008,757, filed on September 24, 2013, entitled, "Navigation System Including Optical and Non-Optical Sensors," hereby incorporated by reference. The navigation system 32 tracks movement of various objects. Such objects include, for example, the manipulator 14, the tool 20 and the anatomy, e.g., femur F and tibia T. The navigation system 32 tracks these objects to gather state information of each object with respect to a (navigation) localizer coordinate system LCLZ. Coordinates in the localizer coordinate system LCLZ may be transformed to the manipulator coordinate system MNPL, and/or vice-versa, using transformations.

The navigation system 32 includes a cart assembly 34 that houses a navigation controller 36, and/or other types of control units. A navigation user interface UI is in operative communication with the navigation controller 36. The navigation user interface includes one or more displays 38. The navigation system 32 can display a graphical representation of the relative states of the tracked objects to the user using the one or more displays 38. The navigation user interface UI further comprises one or more input devices to input information into the navigation controller 36 or otherwise to select/control certain aspects of the navigation controller 36. Such input devices include interactive touchscreen displays. However, the input devices may include any one or more of: push buttons, a keyboard, a mouse, a microphone (voice-activation), gesture control devices, and the like.

The navigation system 32 also includes a localizer 44 coupled to the navigation controller 36. In one example, the localizer 44 is an optical localizer and includes a camera unit 46. The camera unit 46 has an outer casing 48 that houses one or more optical sensors 50. The localizer 44 may comprise its own localizer controller 49 and may further comprise a video camera VC.

The navigation system 32 includes one or more trackers. In one example, the trackers include a pointer tracker PT, one or more manipulator trackers 52A, 52B, a first patient tracker 54, and a second patient tracker 56. In the illustrated example of Figure 1, the manipulator tracker is rigidly attached to the tool 20 (i.e., tracker 52A), the first patient tracker 54 is firmly affixed to the femur F of the patient 12, and the second patient tracker 56 is firmly affixed to the tibia T of the patient 12. In this example, the patient trackers 54, 56 are firmly affixed to sections of bone. The pointer tracker PT is firmly affixed to a pointer or probe P utilized for registering the anatomy to the localizer coordinate system LCLZ. The manipulator tracker 52A, 52B may be affixed to any suitable component of the manipulator 14, in addition to, or other than the tool 20, such as the base 16 (i.e., tracker 52B), or any one or more links 18 of the manipulator 14. The trackers 52A, 52B, 54, 56, PT may be fixed to their respective components in any suitable manner. For example, the trackers may be rigidly fixed, flexibly connected (optical fiber), or not physically connected at all (ultrasound), as long as there is a suitable (supplemental) way to determine the relationship (measurement) of that respective tracker to the object that it is associated with.

Any one or more of the trackers may include active markers 58. The active markers 58 may include light emitting diodes (LEDs). Alternatively, the trackers 52A, 52B, 54, 56, PT may have passive markers, such as reflectors, which reflect light emitted from the camera unit 46. Other suitable markers not specifically described herein may be utilized.

The localizer 44 tracks the trackers 52A, 52B, 54, 56, PT to determine a state of each of the trackers 52A, 52B, 54, 56, PT, which correspond respectively to the state of the object respectively attached thereto. The localizer 44 may perform known triangulation techniques to determine the states of the trackers 52, 54, 56, PT, and associated objects. The localizer 44 provides the state of the trackers 52A, 52B, 54, 56, PT to the navigation controller 36. In one example, the navigation controller 36 determines and communicates the state the trackers 52A, 52B, 54, 56, PT to the manipulator controller 26. As used herein, the state of an object includes, but is not limited to, data that defines the position and/or orientation of the tracked object or equivalents/derivatives of the position and/or orientation. For example, the state may be a pose of the object, and may include linear velocity data, and/or angular velocity data, and the like.

The navigation controller 36 may comprise one or more computers, or any other suitable form of controller. Navigation controller 36 may have a central processing unit (CPU), graphics processing unit (GPU) and/or other processors, non-transitory memory, and storage. The processors can be any type of processor, microprocessor, or multi-processor system. The navigation controller 36 is loaded with software. The software, for example, converts the signals received from the localizer 44 into data representative of the position and orientation of the objects being tracked. The navigation controller 36 may additionally, or alternatively, comprise one or more microcontrollers, field programmable gate arrays, systems on a chip, discrete circuitry, and/or other suitable hardware, software, or firmware that is capable of conducting the functions described herein. The term processor is not intended to limit any embodiment to a single processor.

Although one example of the navigation system 32 is shown that employs triangulation techniques to determine object states, the navigation system 32 may have any other suitable configuration for tracking the manipulator 14, tool 20, and/or the patient 12.

In another example, the navigation system 32 and/or localizer 44 are ultrasound-based. For example, the navigation system 32 may comprise an ultrasound imaging device coupled to the navigation controller 36. The ultrasound imaging device images any of the aforementioned objects, e.g., the manipulator 14, the tool 20, and/or the patient 12, and generates state signals to the navigation controller 36 based on the ultrasound images. The ultrasound images may be 2-D, 3-D, or a combination of both. The navigation controller 36 may process the images in near real-time to determine states of the objects. The ultrasound imaging device may have any suitable configuration and may be different than the camera unit 46 as shown in Figure 1.

In another example, the navigation system 32 and/or localizer 44 are radio frequency (RF)-based. For example, the navigation system 32 may comprise an RF transceiver coupled to the navigation controller 36. The manipulator 14, the tool 20, and/or the patient 12 may comprise RF emitters or transponders attached thereto. The RF emitters or transponders may be passive or actively energized. The RF transceiver transmits an RF tracking signal and generates state signals to the navigation controller 36 based on RF signals received from the RF emitters. The navigation controller 36 may analyze the received RF signals to associate relative states thereto. The RF signals may be of any suitable frequency. The RF transceiver may be positioned at any suitable location to track the objects using RF signals effectively. Furthermore, the RF emitters or transponders may have any suitable structural configuration that may be much different than the trackers 52A, 52B, 54, 56, PT shown in Figure 1.

In yet another example, the navigation system 32 and/or localizer 44 are electromagnetically based. For example, the navigation system 32 may comprise an EM transceiver coupled to the navigation controller 36. The manipulator 14, the tool 20, and/or the patient 12 may comprise EM components attached thereto, such as any suitable magnetic tracker, electro-magnetic tracker, inductive tracker, or the like. The trackers may be passive or actively energized. The EM transceiver generates an EM field and generates state signals to the navigation controller 36 based upon EM signals received from the trackers. The navigation controller 36 may analyze the received EM signals to associate relative states thereto. Again, such navigation system examples may have structural configurations that are different than the navigation system 32 configuration shown in Figure 1.

The navigation system 32 may have any other suitable components or structure not specifically recited herein. Furthermore, any of the techniques, methods, and/or components described above with respect to the navigation system 32 shown may be implemented or provided for any of the other examples of the navigation system 32 described herein. For example, the navigation system 32 may utilize solely inertial tracking or any combination of tracking techniques, and may additionally or alternatively comprise, fiber opticbased tracking, machine-vision tracking, and the like.

The pointer P may be hand-held or may optionally be coupled to the adjustable arm (AA), as shown in FIG. 1. The pointer P may include a force/torque sensor S' that is configured to measure forces/torques applied to the pointer tip. The pointer P may also include a controller, e.g., housed within the pointer body to process measurements from the force/torque sensor S.' Measurements from the pointer P may be communicated to the control system 60 using a wired or wireless connection.

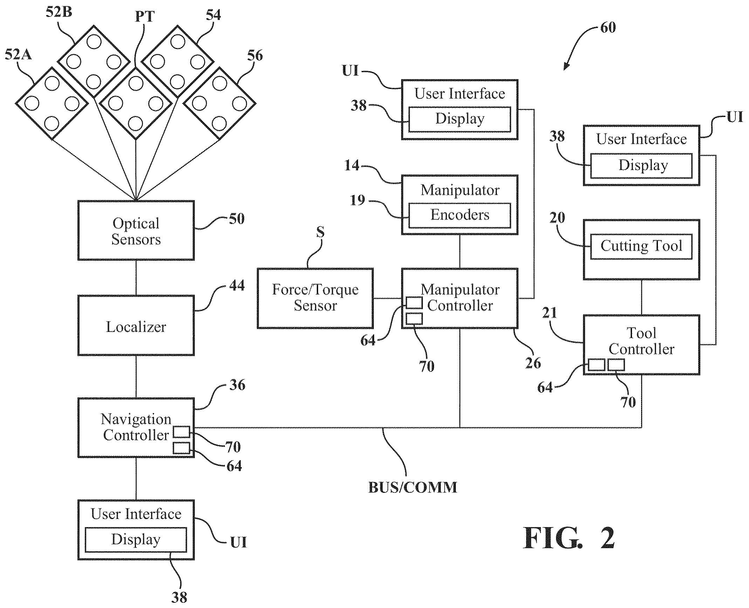

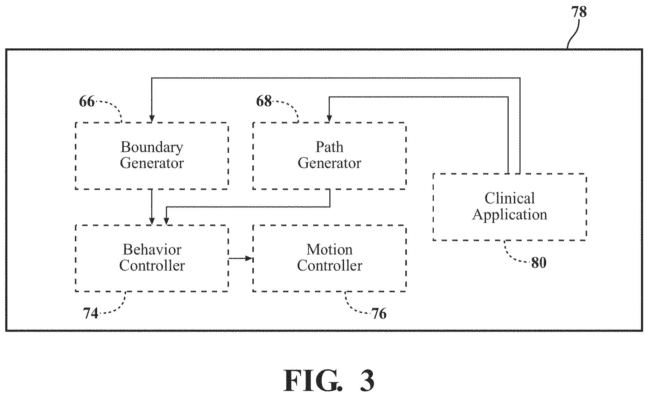

Referring to Figure 2, the surgical system 10 includes the control system 60 that comprises, among other components, the manipulator controller 26, the navigation controller 36, and the tool controller 21. The control system 60 further includes one or more software programs and software modules shown in Figure 3. The software modules may be part of the program or programs that operate on the manipulator controller 26, navigation controller 36, tool controller 21, or any combination thereof, to process data to assist with control of the surgical system 10. The software programs and/or modules include computer readable instructions stored in non-transitory memory 64 on the manipulator controller 26, navigation controller 36, tool controller 21, or a combination thereof, to be executed by one or more processors 70 of the controllers 21, 26, 36. The memory 64 may be any suitable configuration of memory, such as RAM, non-volatile memory, etc., and may be implemented locally or from a remote database. Additionally, software modules for prompting and/or communicating with the user may form part of the program or programs and may include instructions stored in memory 64 on the manipulator controller 26, navigation controller 36, tool controller 21, or any combination thereof. The user may interact with any of the input devices of the navigation user interface UI or other user interface UI to communicate with the software modules. The user interface software may run on a separate device from the manipulator controller 26, navigation controller 36, and/or tool controller 21.

The control system 60 may comprise any suitable configuration of input, output, and processing devices suitable for conducting the functions and methods described herein. The control system 60 may comprise the manipulator controller 26, the navigation controller 36, or the tool controller 21, or any combination thereof, or may comprise only one of these controllers. These controllers may communicate via a wired bus or communication network as shown in Figure 2, via wireless communication, or otherwise. The control system 60 may also be referred to as a controller. The control system 60 may comprise one or more microcontrollers, field programmable gate arrays, systems on a chip, discrete circuitry, sensors, displays, user interfaces, indicators, and/or other suitable hardware, software, or firmware that is capable of performing the functions described herein.

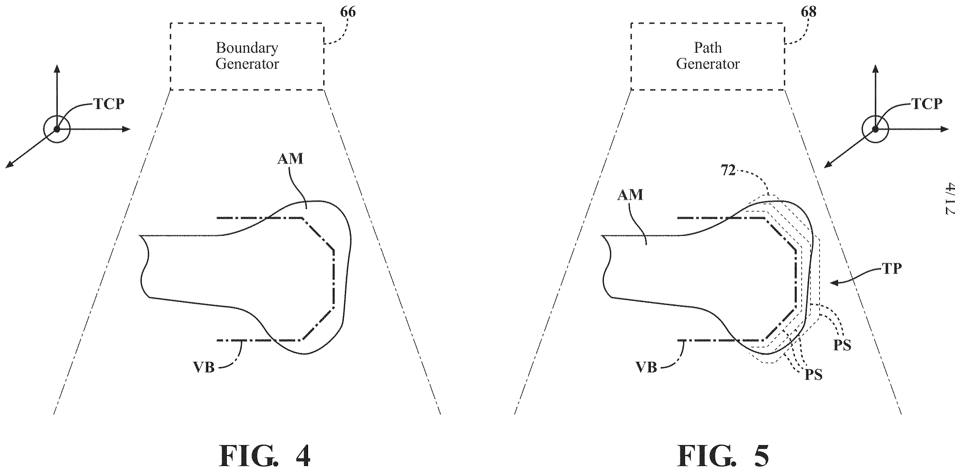

Referring to Figure 3, the software employed by the control system 60 includes a boundary generator 66. As shown in Figure 4, the boundary generator 66 is a software program or module that generates a virtual boundary VB for constraining movement and/or operation of the tool 20. The virtual boundary VB may be one-dimensional, twodimensional, three-dimensional, and may comprise a point, line, axis, trajectory, plane, or other shapes, including complex geometric shapes. In some embodiments, the virtual boundary VB is a surface defined by a triangle mesh. Such virtual boundaries VB may also be referred to as virtual objects. The virtual boundaries VB may be defined with respect to an anatomical model AM, such as a 3-D bone model. In the example of Figure 4, the virtual boundaries VB are planar boundaries to delineate five planes for a total knee implant, and are associated with a 3-D model of the head of the femur F. The anatomical model AM is registered to the one or more patient trackers 54, 56 such that the virtual boundaries VB become associated with the anatomical model AM. The virtual boundaries VB may be implant-specific, e.g., defined based on a size, shape, volume, etc. of an implant and/or patient-specific, e.g., defined based on the patient's anatomy. The virtual boundaries VB may be boundaries that are created pre-operatively, intra-operatively, or combinations thereof. In other words, the virtual boundaries VB may be defined before the surgical procedure begins, during the surgical procedure (including during tissue removal), or combinations thereof. In any case, the control system 60 obtains the virtual boundaries VB by storing/retrieving the virtual boundaries VB in/from memory, obtaining the virtual boundaries VB from memory, creating the virtual boundaries VB pre-operatively, creating the virtual boundaries VB intra-operatively, or the like.

The manipulator controller 26 and/or the navigation controller 36 track the state of the tool 20 relative to the virtual boundaries VB. In one example, the state of the TCP is measured relative to the virtual boundaries VB for purposes of determining haptic forces to be applied to a virtual rigid body model via a virtual simulation 88 so that the tool 20 remains in a desired positional relationship to the virtual boundaries VB (e.g., not moved beyond them). The results of the virtual simulation 88 are commanded to the manipulator 14. The control system 60 controls/positions the manipulator 14 in a manner that emulates the way a physical handpiece would respond in the presence of physical boundaries/barriers. The boundary generator 66 may be implemented on the manipulator controller 26. Alternatively, the boundary generator 66 may be implemented on other components, such as the navigation controller 36.

Referring to Figures 3 and 5, a path generator 68 is another software program or module run by the control system 60. In one example, the path generator 68 is run by the manipulator controller 26. The path generator 68 generates a tool path TP for the tool 20 to traverse. The tool path TP may comprise a plurality of path segments PS, or may comprise a single path segment PS. The path segments PS may be straight segments, curved segments, combinations thereof, or the like. The tool path TP may be defined with respect to the manipulator 14 coordinate system MNPL, localizer coordinate system LCLZ, coordinate system of the tool 20, coordinate system of the anatomy, or any combination thereof. The tool path TP can be virtually attached to the coordinate system of the respective object such that if the object were to move, the tool path TP will correspondingly move. The tool path TP may be implant-specific, e.g., defined based on a size, shape, volume, etc. of an implant and/or patient-specific, e.g., defined based on the patient's anatomy. The tool path TP can be associated with a virtual model of the anatomy and the virtual model and tool path can be registered to the anatomy using the navigation system 32. The control system 60 can generate or obtain the tool path TP by storing/retrieving the tool path TP in/from memory, creating the tool path TP pre-operatively, creating the tool path TP intra-operatively, or the like. The tool path TP may have any 3D shape, or combinations of shapes, such as circular, helical/corkscrew, linear, curvilinear, combinations thereof, and the like.

In one implementation, the tool path TP is defined as a guidance or alignment path. In one example, the tool path TP is for guiding the tool 20 to move to a location that positions the tool 20 for a start of the surgical procedure, or step. For instance, if the tool 20 is a saw blade, the tool path TP may be configured to guide the saw blade to align to a cut plane associated with the anatomy. If the tool 20 is a cutting bur, the tool path TP may be configured to guide the cutting bur to a starting point in preparation for automated cutting. A lead-in path could be virtually connected from the starting point to another cutting path for removal of tissue. The tool path TP may also enable the tool 20 to move along a predefined path of motion for purposes of registering components of the manipulator 14 to the navigation system 32. The tool path TP can be registered to the anatomy using the navigation system 32 such that the tool path TP is virtually fixed to the anatomy. This way, the tool path TP location in space will automatically be updated to account for any movement of the anatomy.

**In** another implementation, as shown in FIG. 5, the tool path TP is defined as a tissue removal path. One example of the tissue removal path described herein comprises a milling path 72. The term "milling path" generally refers to the path of the tool 20 in the vicinity of the target site for milling the anatomy and is not intended to require that the tool 20 be operably milling the anatomy throughout the entire duration of the path. For instance, as will be understood in further detail below, the milling path 72 may comprise sections or segments where the tool 20 transitions from one location to another without milling. Additionally, other forms of tissue removal along the milling path 72 may be employed, such as tissue ablation, and the like. The milling path 72 may be a predefined path that is created pre-operatively, intra-operatively, or combinations thereof. In other words, the milling path 72 may be defined before the surgical procedure begins, during the surgical procedure (including during tissue removal), or combinations thereof.

One example of a system and method for generating the virtual boundaries VB and/or the milling path 72 is described in U.S. Patent No. 9,119,655, entitled, "Surgical Manipulator Capable of Controlling a Surgical Instrument in Multiple Modes," the disclosure of which is hereby incorporated by reference. In some examples, the virtual boundaries VB and/or tool paths TP may be generated offline rather than on the manipulator controller 26 or navigation controller 36. Thereafter, the virtual boundaries VB and/or tool paths TP may be utilized at runtime by the manipulator controller 26.

Referring to Figure 3, two additional software programs or modules run on the manipulator controller 26 and/or the navigation controller 36. One software module is a behavior controller 74. Behavior controller 74 can compute data that indicates the next commanded pose and/or orientation (e.g., pose) for the tool 20. In some cases, only the position of the TCP is output from the behavior controller 74, while in other cases, the position and orientation of the tool 20 is output. Output from the boundary generator 66, the path generator 68, and a force/torque sensor S may feed as inputs into the behavior controller 74 to determine the next commanded pose and/or orientation for the tool 20. The behavior controller 74 may process these inputs, along with one or more virtual constraints described further below, to determine the commanded pose. The behavior controller 74 can be implemented in an admittance control mode, wherein the robotic surgical system proactively generates the commanded position based on an output of a virtual rigid body simulation.

The second software module can include a motion controller 76. One aspect of motion control is the control of the manipulator 14. The motion controller 76 receives data defining the next commanded pose from the behavior controller 74. Based on these data, the motion controller 76 determines the next position of the joint angles of the joints J of the manipulator 14 (e.g., via inverse kinematics and Jacobian calculators) so that the manipulator 14 is able to position the tool 20 as commanded by the behavior controller 74, e.g., at the commanded pose. In other words, the motion controller 76 processes the commanded pose, which may be defined in Cartesian space, into joint angles of the manipulator 14, so that the manipulator controller 26 can command the joint motors accordingly, to move the joints J of the manipulator 14 to commanded joint angles corresponding to the commanded pose of the tool 20. In one version, the motion controller 76 regulates the joint angle of each joint J and continually adjusts the torque that each joint motor outputs to, as closely as possible, ensure that the joint motor drives the associated joint J to the commanded joint angle.

The boundary generator 66, path generator 68, behavior controller 74, and motion controller 76 may be sub-sets of a software program 78. Alternatively, each may be software programs that operate separately and/or independently in any combination thereof. The term "software program" is used herein to describe the computer-executable instructions that are configured to perform the various capabilities of the technical solutions described. For simplicity, the term "software program" is intended to encompass, at least, any one or more of: the boundary generator 66, path generator 68, behavior controller 74, and/or motion controller 76. The software program 78 can be implemented on the manipulator controller 26, navigation controller 36, or any combination thereof, or may be implemented in any suitable manner by the control system 60.

A clinical application 80 may be provided to manage user interaction. The clinical application 80 handles many aspects of user interaction and coordinates the surgical workflow, including pre-operative planning, implant placement, registration, bone preparation visualization, and post-operative evaluation of implant fit, etc. The clinical application 80 is configured to output to the displays 38. The clinical application 80 may run on its own separate processor or may run alongside the navigation controller 36. In one example, the clinical application 80 interfaces with the boundary generator 66 and/or path generator 68 after implant placement is set by the user, and then sends the virtual boundary VB and/or tool path TP returned by the boundary generator 66 and/or path generator 68 to the manipulator controller 26 for execution. Manipulator controller 26 executes the tool path TP as described herein. The manipulator controller 26 may additionally create certain segments (e.g., lead-in segments) when starting or resuming machining to smoothly get back to the generated tool path TP. The manipulator controller 26 may also process the virtual boundaries VB to generate corresponding virtual constraints as described further below.

The surgical system 10 may operate in a manual mode, such as described in U.S. Patent No. 9,119,655, incorporated herein by reference. Here, the user manually directs, and the manipulator 14 executes movement of the tool 20 and its energy applicator 24 at the surgical site. The user physically contacts the tool 20 to cause movement of the tool 20 in the manual mode. In one version, the manipulator 14 monitors forces and torques placed on the tool 20 by the user to position the tool 20. For example, the manipulator 14 may comprise the force/torque sensor S that detects the forces and torques applied by the user and generates corresponding input utilized by the control system 60 (e.g., one or more corresponding input/output signals). In some implementations, the user may be required to continually grasp a trigger or switch on the end effector 22 to enable the force/torque sensor S that detects the forces and torques applied by the user.

The force/torque sensor S may comprise a 6-DOF force/torque transducer. The manipulator controller 26 and/or the navigation controller 36 receives the input (e.g., signals) from the force/torque sensor S. In response to the user-applied forces and torques, the manipulator 14 moves the tool 20 in a manner that emulates the movement that would have occurred based on the forces and torques applied by the user. Movement of the tool 20 in the manual mode may also be constrained in relation to the virtual boundaries VB generated by the boundary generator 66. In some versions, measurements taken by the force/torque sensor S are transformed from a force/torque coordinate system FT of the force/torque sensor S to another coordinate system, such as a virtual mass coordinate system VM in which the virtual simulation 88 is carried out on the virtual rigid body model of the tool 20 so that the forces and torques can be virtually applied to the virtual rigid body in the virtual simulation 88 to ultimately determine how those forces and torques (among other inputs) would affect movement of the virtual rigid body, as described below.

The surgical system 10 may also operate in a semi-autonomous or automated mode in which the manipulator 14 moves the tool 20 along the milling path 72 (e.g., the active joints J of the manipulator 14 operate to move the tool 20 without requiring force/torque on the tool 20 from the user). An example of operation in the automated mode is also described in U.S. Patent No. 9,119,655, incorporated herein by reference. In some embodiments, when the manipulator 14 operates in the automated mode, the manipulator 14 is capable of moving the tool 20 free of user applied forces. In other words, the user does not need to physically contact the tool 20 to move the tool 20. Instead, the user may use some form of remote control to control starting and stopping of movement. For example, the user may hold down a button of the remote control to start movement of the tool 20 and release the button to stop movement of the tool 20.

The surgical system 10 may also operate in a guided-manual mode, as described in U.S Patent Application Publication No. US 2020/0281676 A1, entitled "Systems and Methods for Controlling Movement of a Surgical Tool Along a Predefined Path", the contents of which are hereby incorporated by reference in their entirety. In the guided-manual mode, the user applies forces/torques to the force/torque sensor S and the applied forces/torques are utilized to determine how far to advance the tool 20 along the tool path TP. In the guided-manual mode, the tool 20 is constrained to the tool path TP in 2DOF normal to the tool path, but unconstrained in 1DOF tangential to the tool path TP. In effect, this enables the tool 20 to freely move along the tool path TP based on manual input, but the constraints guide the user by restricting the manual movement of the tool 20 to be along the tool path.

The techniques described herein can utilize constraint equations and data, forward dynamics algorithms, rigid body calculations, constraint force calculations, and virtual simulations like those described in U.S Patent Application Publication No. US 2020/0281676 A1, entitled "Systems and Methods for Controlling Movement of a Surgical Tool Along a Predefined Path", the contents of which are hereby incorporated by reference in their entirety.

### II. Example Manipulator Control Scheme

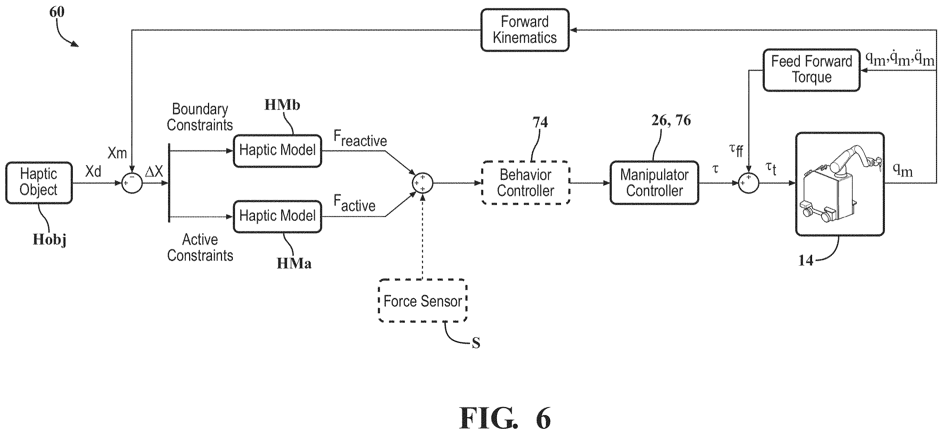

Referring to FIG 6, described in this section is an example control scheme implemented by the control system 60 for controlling the manipulator 14 using haptic forces and joint torques. In one implementation, the control system 60 is configured to implement constraints on the manipulator 14 or the surgical tool 20 pursuant to predefined virtual fixtures or haptic objects (Hobj). Although the robot control problem varies for each type of surgical procedure or tool, these constraints can be divided into two subspaces i.e., active constraints and boundary constraints. The active and boundary constraints impose "task space" constraints on movement of the surgical tool 20. The task space is the Cartesian space defined by the task the manipulator 14 is performing. The task space can be defined by the set of all possible poses of the surgical tool 20 for the given task. The dimensions of the task space will depend on the surgical procedure, step of the procedure, type, or surgical tool 20, or the like. For example, tasks in robotics-assisted arthroplasty require up to six DOF of manipulator control depending on the type of the resection and the surgical tool 20 used for that resection. As an example, total knee arthroplasty (TKA) saw cutting can be a fully constrained task which requires the saw blade to be fully controlled in all six DOFs. Screw/post placement task in shoulder and spine surgery may require a burring attachment with robot control in five DOF (roll motion control is not required during burring). The task space for robotic surgery may be defined by a coordinate system of the implant, patient, or robot, or combinations thereof.

Active constraints attempt to actively virtually constrain specified DOF(s) of the surgical tool 20. Active constraints provide the surgeon with resistance or guidance of the surgical tool 20 by actively restricting a surgeon's movement of the surgical tool in a specified manner. For example, active constraints can constrain certain DOF of the surgical tool so that the surgical tool will feel stiff if the surgeon attempts to move the tool in one of the constrained DOF. Such active constraints can be imposed in any of the described operating modes of the manipulator 14, i.e., manual mode, automated mode, guided-manual mode, etc. It is not necessary that the surgeon interact with the tool 20 to impose the active constraints.

Boundary constraints are used to provide a boundary on movement of the surgical tool 20, e.g., to keep the surgical tool in a zone or keep the surgical tool out of a zone. The boundary constraints are the above-describe virtual boundaries VB that are generated by the boundary generator 66. For the boundary constraints, a reaction force is generated in response to contact or potential contact of the tool 20 to the virtual boundary VB. Such boundary constraints can be imposed in any of the described operating modes of the manipulator 14, i.e., manual mode, automated mode, guided-manual mode, etc.

As shown in FIG. 6, the control system 60 may be configured to implement a haptic model (HMb) for computing haptic forces to implement boundary constraints and a haptic model (HMa) for computing haptic forces to implement active constraints. These haptic models (HMa, HMb) can be separate or combined. When separate, as shown, one or more haptic object(s) (Hobj) is inputted into each haptic model (HMa, HMb). The haptic object (Hobj) may be the same or different for each model. Either of these haptic models can be implemented using any suitable control scheme, including but not limited to: a spring-damper model; a proportional-derivative (PD) model; or an impulse model. The active constraint haptic model (HMa) generates haptic forces (F_{active}) that are intended to actively constrain specified DOF of the surgical tool, i.e., pursuant to the haptic object definition. The boundary constraint haptic model (HMb) generates reactive haptic forces (F_{reactive}) that are configured to reduce interaction between the surgical tool 20 and the virtual boundary VB or haptic object geometry if such interaction is present or imminent. Impulse modeling can be used to compute reactive haptic forces without requiring boundary penetration.

Numerous examples of active and boundary constraints are contemplated. For boundary constraints, the haptic objects (Hobj) can be implemented with a variety of shapes including some predefined geometric constraints such as a plane, a line, or a volume (e.g., cylinder, cone, or box) haptic. Haptic object shape can be also defined more generically using a polygon mesh constraint which is composed of a set of triangles that are connected by their common edges and vertices. For example, the haptic object (Hobj) for the boundary constraint can be implemented as a line haptic defined by a mesh volume.

For active constraints, the haptic objects (Hobj) can be implemented by DOF restrictions on the surgical tool 20, which can mimic an intended haptic geometry. In one example, the haptic object (HobJ) for the active constraint is a haptic plane and the haptic control model (HMa) is configured to compute haptic forces (F_{active}) that are intended to constrain at least three specified DOF of the surgical tool 20 relative to the haptic plane. In this case, the at least three specified DOF comprise two rotational DOF (Rx, Ry) and at least one translational DOF (Tz). This way, the tool 20 is allowed to translate in and out of the plane (Tx), translate left or right in the plane (Ty), and rotate within the plane (Rz), while respecting the 3DOF of the planar constraint (Tz, Rx, Ry). Such planar constraints may be suitable for constraining a saw blade during resection, for example. Notably, the planar constraint can be up to 6DOF. The planar constraint may require less DOF when the robotic system employs a passive joint or planarly extending joint.

The haptic object (HobJ) for the active constraint can also be a haptic line. The haptic control model (HMa) is configured to compute the haptic forces (F_{active}) that are intended to constrain at least four specified DOF of the surgical tool 20 relative to the haptic line. The at least four specified DOF can include at least two rotational DOF (Ry, Rz) and two translational DOF (Ty, Tz). This way, the tool 20 is allowed to translate up/down the haptic line (Tx) and rotate (about the tool axis, Rx) corresponding to the haptic line, while respecting the 4DOF of the line constraint (Ty, Tz, Ry, Rz). Similarly, the line constraint can be more restrictive, if desired, e.g., up to 6DOF. In the case in which the active constraint is implemented by a haptic volume, the haptic control model (HMa) is configured to compute haptic forces (F_{active}) intended to constrain up to three specified DOF of the surgical tool 20 relative to the haptic volume. For example, up to three rotational DOF can be constrained. Notably, the planar constraint can be up to 6DOF. Such line constraints may be suitable for constraining a cutting bur, router, drill, screwdriver, or any other tool with a straight shaft, for example.

The haptic forces can be combined to generate a total haptic force. If the described force/torque sensor (S) is utilized (as shown in FIG. 6), the force/torque values obtained from the sensor (S) can be combined with the total haptic force to generate a total force. Based on the total force, the control system 60 (or manipulator controller 26 and/or motion controller 76) is configured to generate commanded joint torques (τ) to control the respective joint(s) (J) of the manipulator 14. If the total force includes active haptic forces (F_{active}), the commanded joint torques will include components to move to constraint poses in attempt to actively constrain the specified DOF of the surgical tool. If the total force includes reactive haptic forces (F_{reactive}), the commanded joint torques will include components to alleviate tool-boundary interaction. If the total force includes forces from the force/torque sensor S, the commanded joint torques will include components to move the surgical tool 20 in a manner that mimics the surgeon's interaction with the surgical tool 20, while respecting the active and boundary constraints. This type of computation can be part of an admittance type system that optionally utilizes the behavior controller 74 to implement a virtual rigid body simulation of the total force to determine commanded positions or joint torques.

The computed joint torque (T) pursuant to the haptic model calculations may optionally be combined with additional feed forward torques (τ_{ff}). Feed forward torques can include gravity torques and forces required for joints to maintain their positions in the specified gravity. Gravity torques and forces can be computed from the actual measured joint positions (qₘ). Feed forward torques can also include joint damping torques to smooth out movements and prevent oscillations of the joints. Damping torques can be calculated from the velocity (q dot m) of the joints. The feed forward torques (τ_{ff}), and the commanded joint torques (τ) from the haptic constraints can be combined into a total commanded torque (τₜ) by which the control system 60 will command the joints. Forward kinematic calculations can be used to determine the measured pose (Xₘ) of the surgical tool 20 or TCP. The measured pose (Xₘ) of the surgical tool 20 can be fed back into the control loop to determine the difference between the measured pose (Xₘ) and the desired pose (X_{d}) of the surgical tool relative to the haptic objects.

The difference of this comparison (ΔX) is used in the next time step to recompute the necessary boundary and active constraints, and this process can repeat for multiple time steps. The processes described herein can be iteratively performed and repeated for any number of time steps during run-time of the manipulator 14 and can do so depending on presence or absence of conditions, such as detected tool-tissue interaction, surgical steps, operation of certain modes of operation (e.g., manual, automated, guided-manual), etc. This way, the machine learning models (MLM) can continue adaptive learning and optimization of the robotic behavior.

### III. Techniques for Employing Machine Learning Models to Characterize Tissue and Tool Interactions

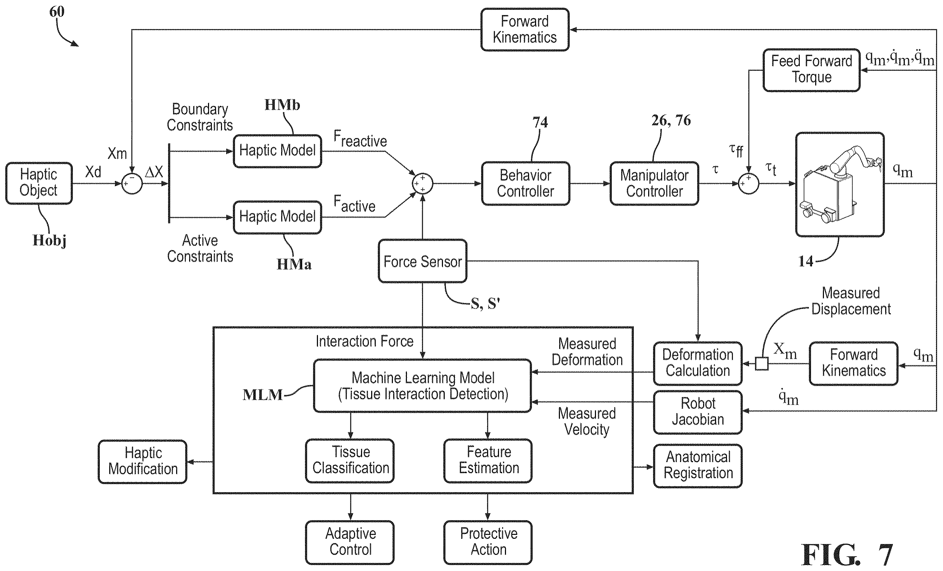

With reference to FIGS. 7-13, described herein are systems, methods, and non-transitory computer readable media (computer program products) for employing machine learning models to characterize interactions between the tool 20 and other objects. As will be described below, such objects can include a wide variety of objects that can be located at the surgical site, such as soft tissue, bone, foreign body objects, or other surgical tools. The techniques also enable characterization of aspects of the objects with which the tool 20 interacts. To briefly summarize, the techniques involve the control system 60 obtaining sensed measurements during interactions of the surgical tool 20 with a surgical site (e.g., tissue/bone) and applying the sensed measurements to one or more machine learning models (MLM) that is/are configured to make a characterization related to the tissue or the tool interactions. Such characterizations can include but are not limited to: object type/subtype classifications; material property characterizations; geometrical feature characterizations; embedded object characterizations; and interaction type characterizations. Based on these characterizations, the manipulator 14 can be controlled in numerous manners, such as to perform tasks such as: anatomical registration, modification of haptics or haptic control, implement protective actions, implement adaptive control, and the like.

The techniques described herein provide numerous technical solutions and advantages. For example, the solutions described address the intricacy of objects/tissues at the surgical site by providing a control scheme that is well-equipped to understand the true nature of the objects with which the tool 20 interacts. The control scheme described herein can adequately recognize and account for the unknown contact dynamics stemming from the tool interactions. Such unknown contact dynamics can result from complex factors, such as the properties of the objects, friction, contact forces, or geometry variations. In turn, the solutions described can characterize the nature of the objects with which the tool interacts or distinguish different object types. The machine learning techniques described herein are dynamic and adaptable, thereby providing a customized control scheme based on real-time environmental interactions and by making intelligent predictions about the contact dynamics occurring between the tool and the objects. As a result, the accuracy of the tool is improved, particularly relative to the surgical site. Tool accuracy improvements will improve performance of the manipulator 14, exhibit more predictive tool behavior, and provide a more optimal surgical outcome for the patient. Moreover, the described machine learning techniques unlock the ability for the manipulator 14 to perform higher order tasks of greater complexity, as will be described below.

### A. Introduction to Machine Learning Model and Inputs

With reference to FIG. 7, an example block diagram is provided illustrating control processes that can be performed by the described techniques to perform the characterization based on the tool interactions. Throughout this description, the steps or processes of the control schemes will be described as being performed by the control system 60. As described, the control system 60 can include any one or more of the described controllers or components of the surgical system. The block diagram of FIG. 7 augments the block diagram of FIG. 6 by including the machine learning model (MLM) configured for the described solutions. The machine learning model (MLM) can be added or incorporated into the control system 60 to refine aspects of the control system 60. In other implementations, the machine learning model (MLM) can be used as a substitute for components/software of the control system 60. For example, the machine learning model (MLM) can substitute for the behavior controller 74, and/or the motion controller 76.

As shown in the diagram and will be described below, the machine learning model (MLM) is configured to receive and process various input measurements, including interaction forces, measured tool displacements (or deformations, if applicable), and measured tool velocities (or deformation velocities, if applicable). The control system 60 can obtain these measurements responsive to interactions of the surgical tool 20 with the surgical site over time.

The surgical tool 20 generally performs some type of action when interacting with the objects at the surgical site. Actions of the tool 20 may be sliding across an object, pressing into the object, and/or palpating the object. Actions of the tool 20 can also be surgical actions, such as cutting, drilling, pointing, sawing, routing, reaming, impacting, milling, sculpting, or any other form or manipulating.

The interaction force(s) are the actual or predicted forces that are applied to the surgical tool 20, e.g., during interaction with the respective objects at the surgical site. The interaction forces can be obtained using various methods. For example, as shown in FIG. 7, the interaction forces can be obtained by the force/torque sensor (S) that is coupled to the surgical tool 20 or the manipulator 14. If the adjustable arm (AA) is utilized, the force sensor (S') of the adjustable arm (AA) or pointer tool (P) attached to the adjustable arm (AA) may be used. Other forms of force sensing can be utilized, such as implementing other force sensors, load cells, strain gauges, or pressure sensors that can be included at or near the TCP of the surgical tool 20. In other instances, the control system 60 can implement a force observer to indirectly infer forces acting on the tool 20 using indirect parameters such as velocity and/or acceleration.

The measured displacement(s) are the actual or predicted displacement(s) of the surgical tool 20 pursuant to interaction with the respective objects at the surgical site. In one example, the control system 60 can compute the measured displacements using robotic data. As shown in FIG. 7, a forward kinematic calculation can be applied to the measured joint positions (qm) from the manipulator 14 to obtain the measured tool poses (Xm). The measured tool poses (Xm) can be compared or evaluated over time to determine displacements of the tool 20. Displacements can be measured using other methods, such as from tracking data obtained the navigation system 32 (e.g., tracking the surgical site and tool), inertial sensors provided on the surgical tool 20, or the like.

The force sensor (S) measurements and the measured tool poses (Xm) (and/or displacements) can be used as variables to determine a deformation indicative of the how much the object deformed pursuant to interaction forces applied by the tool 20. The deformation can be actual or predicted. The computation of deformation may be appropriate in circumstances where the respective object is pliable/deformable, such as soft tissue. However, computing deformation need not be required, particularly if the tool is interacting with hard objects, such as bone or metals, in which case, the displacement of the tool may suffice instead of deformation. In one example, the measured deformation can be calculated by measuring the displacement of the surgical tool 20 between an initial and a final point of contact relative to the object. These initial and final points of contact can be identified using the force sensor (S), the navigation system 32, or combinations thereof. Deformation can be measured using other methods, such as from tracking data obtained the navigation system 32 (e.g., tracking the surgical site and tool), inertial sensors provided on the surgical tool 20 and on the tissue, or the like.

The measured velocities are actual or predicted velocities of the surgical tool 20 that are measured during interaction with the respective object at the surgical site. In some cases, the derivative of the displacement can be utilized to compute the velocity. When used in combination with displacement/deformation, the measured velocities can be indicative of measured deformation velocities (or rate of deformation change) of the tissue responsive to forces applied to the tissue by the surgical tool. In one example, as shown in FIG. 7, the measured velocities can be determined based on the measured joint velocities (qm) of the manipulator 14. A Jacobian transform can be used to mathematically relate the measured joint velocities (q̇m) to the measured velocity of the tool 20. Tool velocities can be measured using other methods, such as from tracking data obtained the navigation system 32, inertial sensors provided on the surgical tool 20, or the like. In other examples, the control system 60 can obtain the measured velocities by computing the derivative of the measured deformation(s), described above.

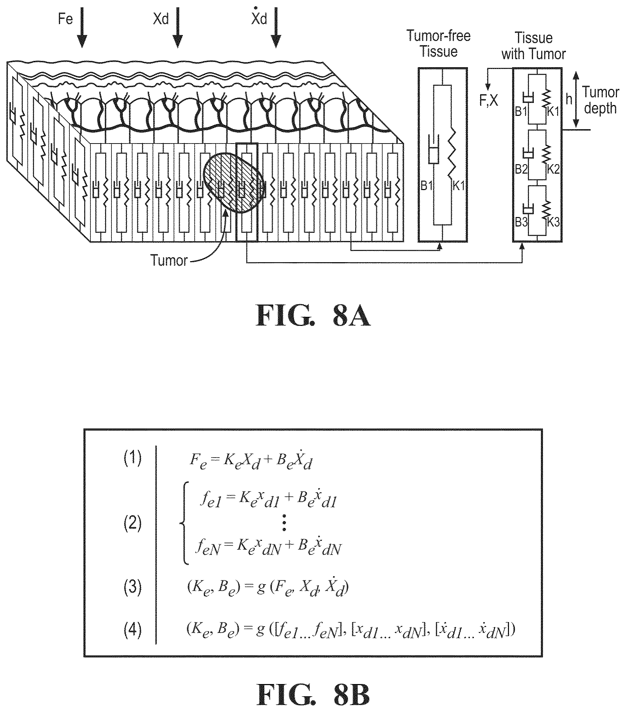

To further illustrate the relationship between the measured interaction forces, displacements and velocities, FIG. 8A illustrates an example spring-damper model of a tissue that includes an embedded tumor. Assuming the tissue exhibits uniform structure, the tissue can be linearly modeled as a spring and damper with a constant stiffness (Ke) and damping (Be). The interaction forces (Fe) applied to the tissue by the tool 20 result in the measured deformation (Xd). The rate of deformation change (Ẋd) (e.g., velocity) can be computed using the derivate of the measured deformation (Xd). With this linear tissue model, there exists a relationship between the applied interaction force (Fe), the measured deformation (Xd) and velocity (Xd) using equation (1) as shown in FIG. 8B. Equation (1) illustrates how the tissue properties can be measured if the force, displacement and the velocity are known. With the linear assumption of the tissue model, the stiffness (Ke) and damping (Be) can be measured if two independent measurements exist, either from two different regions or at two different instants from the same region.

Notably, the surgical system 10 can employ a "sensing system" to obtain any of the described measurements. The sensing system can include any suitable component, sensor, and controllers described above. For example, the sensing system can include, but is not limited to: the control system 60 and any of the described controllers (26, 74, 76), the force sensor (S, S'), tracking data (position, orientation, velocity) from the navigation system 32, robotic data from the manipulator (e.g., joint positions, velocities, accelerations), joint torque values, electrical current drawn from the joint motors, joint position data from the adjustable arm (AA), or any other supplemental sensor(s), such as inertial sensors (on the robot, tool or tissue), pressure or force sensors (near the TCP of the tool), tissue sensors (e.g., strain gauges, optical sensors, capacitive sensors, etc.), and the like.

Any of the techniques described herein for characterizing or classification objects or interactions can be used in any of the described control modes of the manipulator 14, including the manual mode, the semi-autonomous or automated mode, the guided-manual mode, or the like. The techniques can also apply with or without activation of any of the described active constraints or boundary constraints.

### B. Machine Learning Tissue Stiffness and/or Damping Estimation

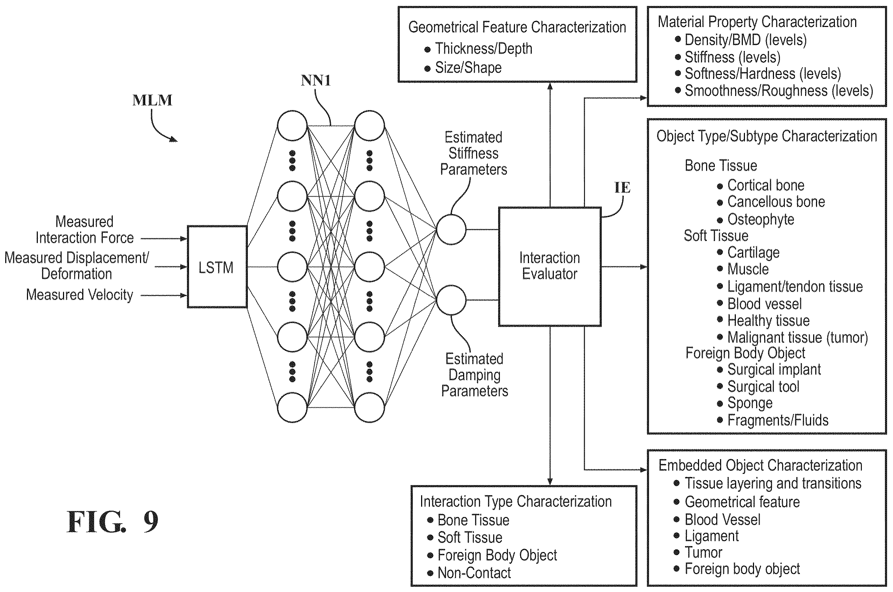

One example architecture of the machine learning model (MLM) is shown with reference to FIG. 9. The machine learning model (MLM) is configured to receive and process the measured values of interaction force(s), displacement(s)/deformation(s), and velocities to estimate stiffness and/or damping parameters of the object pursuant to interactions by the surgical tool 20.

The stiffness parameters are indicative of the object's resistance to deformation under the applied load of the interaction force (e.g., how much the object pushes back on the tool). The damping parameters are indicative of the object's ability to absorb or dissipate energy responsive to the applied load of the interaction force (e.g., how quickly the object loses energy under deformation). The stiffness parameters may be measured using any suitable value, such as Young's modulus, wherein the greater the stiffness, the harder the tissue, and vice versa. The damping parameters may be measured using any suitable damping coefficient, wherein the higher the damping, the faster the object loses energy responsive to the applied load.

The described linear tissue model of FIG. 8A included a constant stiffness (Ke) and damping (Be) and was used to illustrate the relationship between interaction force, displacement/deformation, and velocity. However, the stiffness and damping parameters of the tissue are much more complex and difficult to derive due to the practical reality that tissue typically exhibits nonlinear behavior and dynamic stiffness/damping. Accordingly, the machine learning model (MLM) is configured to intelligently predict non-linear and dynamic stiffness/damping parameters of the tissue. Notably, although stiffness and damping provide synergetic analysis when combined, the machine learning model (MLM) can be configured to estimate stiffness parameters of the tissue (without damping) or estimate damping (without stiffness).

To implement this capability, the machine learning model (MLM) may be configured using a first neural network (NN1). The first neural network (NN1) can include an input layer, hidden layers, and an output layer. The input layer of the first neural network (NN1) can receive the measured values of interaction force(s), displacement(s)/deformation(s), and velocities. The output layer of the first neural network (NN1) can output the estimated stiffness and damping parameters.

The machine learning model (MLM) may be configured to output the stiffness/damping parameters in various manners. As shown in FIG 9, for instance, the output layer of the first neural network (NN1) may include two nodes, one to output the estimated stiffness parameters and one to output the estimated damping parameters. However, it is contemplated that the output layer may include one node to output both the stiffness and damping parameters (combined), only the stiffness parameters, or only the damping parameters. Any node, over time, could output different parameter values. For example, the values of stiffness/damping may change over time. Also, it is possible that one node may output a parameter while the other does not. The output layer may include any suitable number of nodes, depending on how the stiffness/damping parameters may need to be estimated. For instance, one node may output a Young's Modulus value of the stiffness parameter while another node may output a value that relates the stiffness parameter to the damping ratio.

The first neural network (NN1) can be any suitable type of neural network, such as but not limited to: an artificial neural network, a deep-learning network, a transfer learning network, a convolutional neural network (CNN), a recurrent neural network (RNN), a multilayer perceptron (MLP), a generative adversarial network (GAN), a feedforward neural network, an encoder network or transformer, or the like.

The machine learning model (MLM) can optionally include or be coupled to a first long short-term memory (LSTM) or recurrent neural network (RNN). The first LSTM/RNN can be coupled to the input layer of the first neural network (NN1). The first LSTM/RNN can receive the measured values over time. The first LSTM/RNN can modify weights and biases to learn long-term dependencies among the measured values and to selectively filter the measured values. The first LSTM/RNN can provide filtered measured values to the input layer of the first neural network (NN1). The LSTM can process the measured values in a configurable time window. In other instances, e.g., when no velocity measurements are obtained or when making more straightforward classifications, the first LSTM/RNN can be omitted or bypassed, and the input data can flow directly into the first neural network (NN1).

Numerous samples of the measured values may be collected over time. For example, the measured values can be collected from N different regions (distributed tactile force) or at N different sample times within the same region (single point force) to estimate the tissue properties. Therefore, with reference to set of equations in (2) of FIG. 8B, *f*_{e1,...}*f*_{eN} are N different interaction force measurements, (x_{d1,...,}x_{dN}) are N different deformation measurements and (Ẋ_{d1,...,} Ẋ_{dN}) are N different velocity measurements. An optimization algorithm can be utilized by the control system 60 to solve this set of equations (2) to estimate the stiffness and damping parameters of the object.

Although, a simple optimization algorithm may result in measuring Ke and Be for certain interaction force and displacement/deformation measurements, the accuracy of the results depends on whether a linear model can be applied to the object of interaction. As described, however, the model of most objects is often not linear. Additionally, the object may not be uniform, and some embedded objects may be also present. To illustrate in the context of tissue, the example of FIG. 8A demonstrates a case where the tissue contains a tumor. The stiffness of the tumor is greater than that of the surrounding healthy tissue and depending on the depth of the tumor relative to the healthy tissue, the measured stiffness and damping during the tissue palpation may significantly vary. The diagram of FIG. 8A further shows a spring-damper model of tissue with different stiffness and damping constants for a tumor versus the surrounding healthy tissue. Now considering both the non-linearity and non-uniformity of the tissue, we can assume a general non-linear form of relationship between the measured force, displacement, velocity, and the stiffness and damping of the tissue as expressed in equation (3). This equation replaces the equation (1) where the linear relationship was assumed among the measurements and the tissue properties. For the set of equations in (2), we can consider equation (4) where the machine learning model (MLM) is utilized to solve for the stiffness Ke and the damping Be.

The LSTM component is responsible for memorizing the N previous samples of force, displacement/deformation and velocity measurements that can be used to compose the input defined in Equation (4). The first neural network (NN1) can implement the nonlinear function (g) in Equation (4). The machine learning model (MLM) can be trained to determine the weights and biases of the first neural network (NN1) based on sample objects with known properties that can be later used for any given measured force, displacement/deformation, and velocity to estimate the stiffness and damping of the given object. The first neural network (NN1) may be trained using deep-learning, supervised learning or semi-supervised learning. In other instances, the first neural network (NN1) may be implemented using unsupervised learning. The training of the first neural network (NN1) can be based on data that is toolspecific, procedure specific, surgical step specific, control mode specific, or the like. Training can also be based on any tissues/objects of various geometrical features, material properties, and types described herein.

Notably, the machine learning model (MLM) generates the estimated stiffness and damping parameters as embeddings of lower dimensionality than the measured values. The numerical or vector representations of the stiffness and damping data can be reduced in dimensionality relative to the measured values to allow the machine learning model (MLM) to more easily learn patterns and relationships, improve computational efficiency, and enable better predictions for unforeseen data. In one example, the measured values have a dimensionality of more than 2 dimensions, and the estimated stiffness and damping parameters have a dimensionality of up to 2 dimensions. Other dimension ranges are possible and are contemplated.

### C. Machine Learning Interaction Evaluator

With continued reference to FIG. 9, the control system 60 and/or machine learning model (MLM) may employ an interaction evaluator (IE). The interaction evaluator (IE) is configured to receive the estimated stiffness and/or damping parameters outputted by the first neural network (NN1) to predict characterizations about the tool 20 interactions. The interaction evaluator (IE) can receive the estimated stiffness and/or damping parameters continuously, during a predefined window of time, or at discrete times or in discrete batches or packets. The interaction evaluator (IE) can monitor changes in the estimated stiffness and/or damping parameters over time. The interaction evaluator (IE) is configured to process the estimated stiffness and/or damping parameters to predict one or more characterization(s) pursuant to the interaction.

By monitoring the stiffness and damping parameters, the machine learning model (MLM) can reveal any of the described object or interaction characterizations. Namely, the stiffness and damping parameters are based on the measured interaction forces, measured displacements/deformations and measured tool velocities. These measurements, when evaluated over time, can exhibit specific (force/displacement/velocity) waveforms, or signals pursuant to the interaction. Since the actions (force, displacement, velocity) of tool 20 are also embedded within the stiffness and damping parameters, the action of the tool 20 can be utilized in the prediction. For instance, when the tool slides across a fine texture, the signal may be different than when the tool slides across a coarse texture, and so on. The techniques described herein can derive characterizations in response to any action of the tool 20. In some cases, the actions of the tool 20 may be random, variable, user defined, or unknown. In other cases, the actions of the tool 20 may be specified, e.g., due to surgical procedure, clinical, and/or control system requirements, etc.

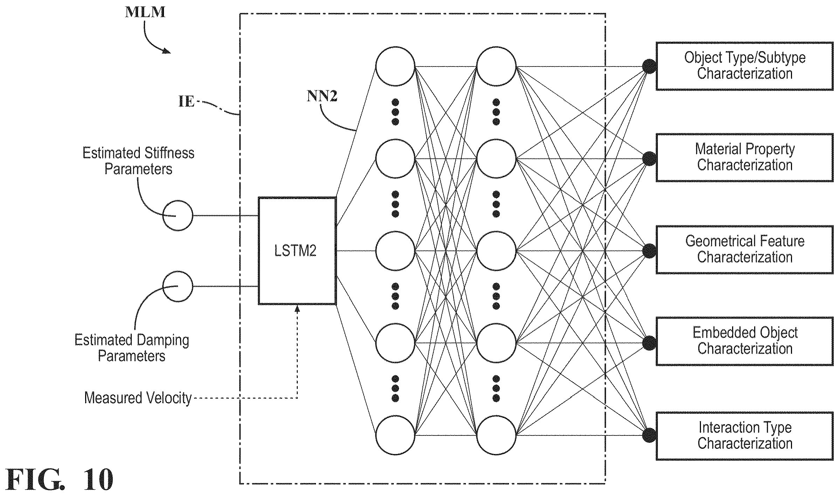

In one implementation, as shown in FIG. 10, the interaction evaluator (IE) is a part of the machine learning model (MLM) and is implemented with a second neural network (NN2). The second neural network (NN2) can include an input layer, hidden layers, and an output layer. The input layer of the second neural network (NN2) can receive the measured estimated stiffness and/or damping parameters. The output layer of the second neural network (NN2) can output the respective characterizations. The second neural network (NN2) can implement a nonlinear function to associate the estimated stiffness/damping parameters to the appropriate characterization(s). The machine learning model (MLM) can be trained to determine the weights and biases of the second neural network (NN2) based on sample stiffness and damping values with known object properties that can be later used for the characterization prediction. The training of the second neural network (NN2) can be based on data that is toolspecific, procedure specific, surgical step specific, control mode specific, or the like. Training can also be based on any tissues/objects of various geometrical features, material properties, and types described herein. The second neural network (NN2) may be trained using deep-learning, supervised learning or semi-supervised learning. In other instances, the second neural network (NN2) may be implemented using unsupervised learning.

The machine learning model (MLM) may be configured to output one or more different characterizations. As shown in FIG. 10, for instance, the output layer of the second neural network (NN2) may include several nodes, each being configured to output a specific type of characterization. For instance, there may be up to five or more nodes. The nodes can be respectively configured to output an object type/subtype classification, material property characterization, geometrical feature characterization, embedded object characterization, and interaction type characterization. Other characterizations are contemplated beyond those shown. Two or more nodes can output values that apply to more than one characterization, which the machine learning model (MLM) can combine to make sophisticated, higher order, predictions about the interaction. Any node, over time, could output characterization values that change over time or change in the type of characterization. Some nodes may output a characterization while others do not. The output layer may include any suitable number of nodes, depending on how many different characterizations may be desired to predict. Moreover, one output node may be configured to generate numerous types of characterizations. Hence, several output nodes are not necessarily required, as shown in FIG. 10.

The second neural network (NN2) can be any suitable type of neural network, such as but not limited to: an artificial neural network, a deep-learning network, a transfer learning network, a convolutional neural network (CNN), a recurrent neural network (RNN), a multilayer perceptron (MLP), a generative adversarial network (GAN), a feedforward neural network, a decoder network or transformer, or the like.

The machine learning model (MLM) can include or be coupled to a second long short-term memory (LSTM2) or recurrent neural network (RNN2). The second LSTM2/RNN2 component is responsible for memorizing the N previous samples of stiffness and/or damping that can be used to determine the respective characterization(s). The second LSTM2/RNN2 can be coupled to the input layer of the second neural network (NN2). The second LSTM2/RNN can receive, over time, the estimated stiffness, and damping parameters from the output layer of the first neural network (NN1). The second LSTM2/RNN2 can modify weights and biases to learn long-term dependencies among the estimated stiffness and damping parameters and to selectively filter the estimated stiffness and damping parameters. The second LSTM/RNN can provide filtered measured values to the input layer of the second neural network (NN2). The second LSTM2/RNN2 can process the estimated stiffness and damping parameters in a configurable time window. In some cases, LSTM2/RNN2 can be configured to only memorize the most recent stiffness and damping parameters predicted by the first neural network (NN1).

Optionally, as shown in FIG. 10, the second LSTM2/RNN2 can further receive, over time, measured values of the velocities. These measured velocity values can be the same or different values that were inputted into the first LSTM1 and first neural network NN1. For example, the measured velocity values can be contemporaneously inputted into both networks, or time-delayed or filtered when inputted into the second LSTM2/RNN2. Input of the measured velocities into the second LSTM2/RNN2 will allow the machine learning model (MLM) to make more complex characterizations, such as, but not limited to: layering characterizations and embedded object size and depth characterizations.

The machine learning model (MLM) can optionally generate the characterization(s) as a vector of greater dimensionality than the estimated stiffness and damping parameters. By doing so, the machine learning model (MLM) can capture more complex relationships that otherwise cannot be known using the lower dimensional stiffness/damping data. In one example, the characterization has a dimensionality of greater than greater than 2 dimensions and the estimated stiffness and damping parameters have a dimensionality of up to 2 dimensions. Other dimension ranges are possible and are contemplated.

In some cases, the machine learning model (MLM) can proactively filter data or reduce its computations in response to the control system 60 providing a general context of the interaction. For instance, if preoperative imaging data reveals that the bone includes no foreign body objects, the machine learning model (MLM) may temporarily filter its second neural network (NN2) or utilize a custom second neural network that omits any characterizations involving foreign body objects.

It is contemplated that the interaction evaluator (IE) can employ a look up table (LUT) that the control system 60 utilizes to correlate the estimated stiffness and/or damping parameters to values related to tissue or interaction characterizations. The look up table can be used in addition to, or instead of the second neural network (NN2) and LSTM2/RNN2.

### D. Characterizations

As described, the machine learning model (MLM) is configured to predict one or more characterizations related to the object with which the tool 20 interacts, or a characterization of the interaction of the tool 20 with the object. By evaluating the stiffness and damping parameters over time, the machine learning model (MLM) can learn attributes (e.g., type, geometrical features, etc.) of the object with which the tool 20 interacts. Such characterizations include, but are not limited to: an object type/subtype classification, material property characterization, geometrical feature characterization, embedded object characterization, and interaction type characterization. A non-exhaustive list of examples for each of these characterizations is provided in the respective blocks of FIG. 9 and will be described below. Notably, the machine learning model (MLM) may be configured to make numerous characterizations in combination. Hence, any of the characterizations described below may be made in combination with any other. Moreover, although the term "characterization" is chosen throughout this document, it should be understood that in certain cases, the term "classification" may be substituted, such as when classifying object types. Furthermore, as the machine learning model (MLM) gains knowledge over time by processing interactions, any of the described characterization will be predicted with greater accuracy.

### 1. Object Type/Subtype Characterization

The machine learning model (MLM) characterization can identify or predict a type of object or subtype of an object. In one example, the object can be the tissue, and the characterization can identify presence of the tissue or a type of tissue. For instance, the tissue type characterization can identify whether the tissue is soft tissue or bone, generally. Additionally, the tissue characterization can also identify a specific subtype of soft tissue or bone. For instance, the tissue characterization can identify soft tissue subtypes, such as cartilage, muscle, ligament tissue, tendon tissue, a blood vessel, healthy tissue, and malignant tissue (or tumor tissue). The tissue characterization can identify bone subtypes, such cortical bone (including inner or outer cortical bone), cancellous bone, osteophytes, etc. Accordingly, the tissue type characterization advantageously can help distinguish materials that a human cannot otherwise readily distinguish, such as osteophytes from cortical bone, or cartilage from cortical bone, and the like.

In other cases, the object may be a foreign body object. Such foreign body objects can include any object that may be located or otherwise found in/on/around the surgical site. Foreign body objects can include, but are not limited to, surgical implants (primary components, trail components, revision components, fasteners, screws, pins, plates), surgical tools (e.g., soft tissue retractors, needles, irrigation tools, aspiration tools, surgical tracker mounts, a surgical camera/scope, a joint tensor/distractor, etc.), surgical sponges, etc. In other cases, the foreign body object can be a metal shard, fragment, fluids (e.g., irrigation/aspirational fluid), or any other known or unknown foreign object. In any case, the machine learning model (MLM) may identify the foreign body object and can distinguish the foreign body object from bone or soft tissue. The foreign body object can also be sub-identified based on its material properties. For instance, the stiffness properties of a metal tool differ from a surgical sponge such that the specific foreign body object may be identified as a surgical tool or sponge.

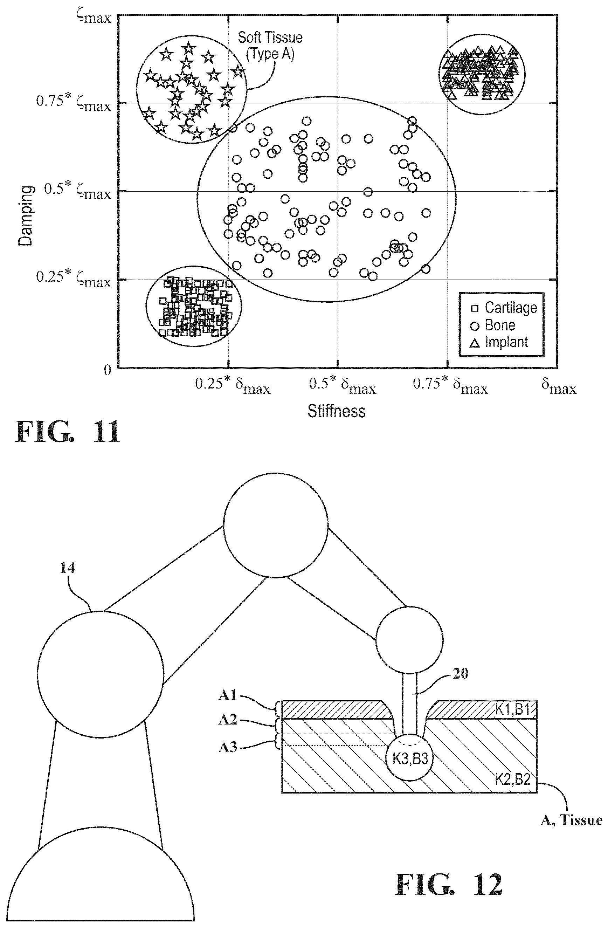

Figure 11 is a plot illustrating sample stiffness and damping parameter values produced by the machine learning model (MLM) over time in effort to characterize various objects at the surgical site. The damping and stiffness parameters are illustrated on their respective axes by a range from 0 to a predefined maximum value of the range. In this specific example, the machine learning model (MLM) learned that cartilage is characterized by stiffness in the range of about 0.1 to 0.25 of the maximum stiffness value and by damping in the range of about 0.1 to 0.25 of the maximum damping value. The bone is characterized by stiffness in the range of about 0.25 to 0.75 of the maximum stiffness value and by damping in the range of about 0.25 to 0.75 of the maximum damping value. Implant material is characterized by stiffness in the range of about 0.75 to the maximum stiffness value and by damping in the range of about 0.75 to the maximum damping value. A soft tissue of a specific type (i.e., type A) is characterized by stiffness in the range of about 0.1 to 0.3 of the maximum stiffness value and by damping in the range of about 0.7 to 0.9 of the maximum damping value. Hence, the machine learning model (MLM) has learned how to distinguish whether the tool is interacting with bone, soft tissue, cartilage, or implant material. Foreign body objects can be classified in a comparable manner. Although not plotted, the machine learning model (MLM) can learn how to classify types or sub-types of any object or material described herein.

### 2. Material Property Characterization

In another example, the machine learning model (MLM) can characterize a material property of the object (e.g., of any of the described objects, such as soft tissue, bone, or foreign body objects). The material properties may include, but are not limited to: density, bone mineral density (BMD), stiffness, hardness, softness, smoothness/fineness, roughness/coarseness, plasticity, elasticity, malleability, compressive strength, or the like. More than one material property may be characterized at the same time or separate times.

The stiffness and damping parameters, when monitored over time, can reveal the material properties of the object. Namely, the stiffness and damping parameters are based on the measured interaction forces, measured displacements/deformations, and measured velocities. These measurements, when evaluated over time, can exhibit specific (force/displacement/velocity) waveforms, or signals pursuant to material properties of the object. Since the actions (force, displacement, velocity) of tool 20 are also embedded within the stiffness and damping parameters, the action of the tool 20 can be utilized in the prediction. For example, when the tool slides across the surface of an object, the signal may exhibit a more linear waveform for the smooth object as compared to a rough object.

The machine learning model (MLM) can characterize the material properties of the object in any suitable manner. For instance, the machine learning model (MLM) can characterize a scale of any material property. The material property may have predefined "levels." For instance, the machine learning model (MLM) may identify density levels I, II, II, etc. Density level I may indicate a T score of 0 or less, density level II may indicate a T score between 0-1 and density level II may indicate a T score greater than +1. In some examples, the properties of the object can be characterized by the amount of stiffness/damping. Any other suitable way of characterizing material properties may be utilized.

### 3. Geometrical Feature Characterization

Another characterization the machine learning model (MLM) can make is a geometrical feature characterization, which defines a geometrical feature of the any of the object subject to interaction. Examples of geometrical features include but are not limited to: a thickness of the object, a depth of the object (relative to reference point), a size of the object (e.g., area, surface area, volume, etc.), a shape of the object, contouring of the object, and other features of interest, such as corners, edges, protrusions, grooves/recesses, etc.

The stiffness and damping parameters, when monitored over time, can reveal the geometrical features of the object. For example, when the tool presses into and slides across a round object, the signal may exhibit a specific shape for the round object as compared to a flat object.

### 4. Interaction Type Characterization

In another example, an interaction type of the tool 20 can be characterized. For instance, the machine learning model (MLM) can characterize the tool interaction being a bone interaction, soft tissue interaction, or even a non-contact interaction (interaction with air). The interaction type may be based, in part, on one of the above characterizations, such as object type characterizations. The interaction type characterization may provide insight different from the characterized object type because the interaction type characterization is aimed at helping the control system 60 understand the current interaction of the tool 20, rather than understanding characteristics of the object itself. For instance, an object may be characterized as tissue (at some point in time), but the tool 20 may interact with bone soon after. In this example, the tissue characterization does not change (because the tissue does not intrinsically change), but the interaction characterization changes because the tool 20 is now interacting with bone. Knowing the interaction type may provide additional insight into other characterizations described above. For instance, by knowing the interaction type is a soft tissue interaction, the machine learning model (MLM) may characterize only soft tissue subtypes.

In some cases, the control system 60 can obtain the interaction type from sources other than the machine learning model (MLM). For instance, robotic data and patient tracking data from the navigation system may be used to determine the distance between the tool 20 and the surgical site. If the tool 20 is spaced away from the surgical site, the interaction is a non-contact interaction, and the machine learning model (MLM) can be provided with this information to facilitate other characterizations. Similarly, the control system 60 can obtain this information from other sources, such as control data/modes, surgical planning information, tool path information, bone model data, or the like.

### 5. Embedded Object Characterization

In some cases, an object may be embedded in another object. For instance, a tumor may be embedded in surrounding soft tissue, a sublayer of tissue may exist below a surface layer of tissue, a portion of an implant may be embedded in bone, etc. The machine learning model (MLM) can characterize features or parameters related to this object embedding. Examples of embedded object characterizations include but are not limited to determining: the layers of tissue; transitions between layers of tissue; the geometrical features of the embedded object; the type of embedded object (e.g., blood vessel, ligament, tumor, foreign body object, etc.), and so on. Such characterizations have many benefits, such as to measure the thickness of cartilage, the size of artery, the presence of an existing implant, the depth of a tumor in the tissue, and the like.

To perform embedded object characterizations, the machine learning model (MLM) can input the measured velocity into the second LSTM2/RNN2, as described above and shown in FIG. 10. Namely, measured velocity (in addition to the stiffness/damping parameters) enables the machine learning model (MLM) to characterize the relationship between the object and its surroundings.

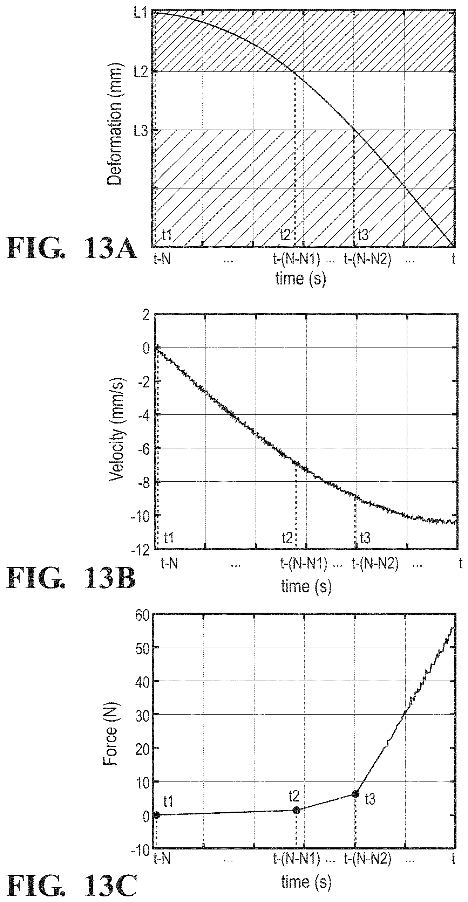

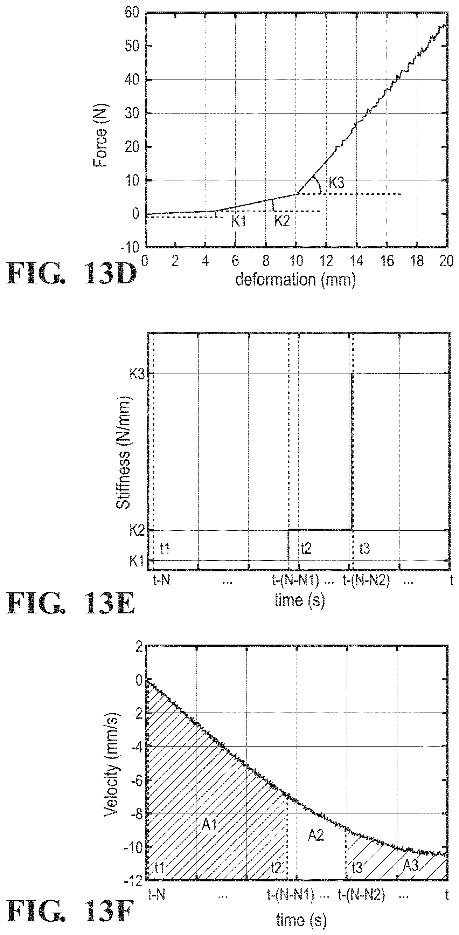

An example of object characterization will now be described with reference to FIGS. 12 and 13. This example is provided only as one of many possible implementations. For the simplicity of description, and to avoid redundancy, it should be appreciated that theory, calculations, measurements, charts, and characterizations described in this example can be used for other characterizations described above.

FIG. 12 is a simplified drawing (with exaggerated scale for illustrative purposes) of a task involving the manipulator 14 and a patient's anatomy (A). The anatomy (A) in this example includes a top layer of tissue with a relatively small stiffness K1 and a second layer of tissue with a stiffness K2 (e.g., assume five times stiffer than the top layer). Furthermore, an embedded object (e.g., tumor or foreign object) with the stiffness K3 (e.g., assume five times stiffer than the second layer of tissue). The objective is to explore how the proposed approach can be utilized to classify these three types of objects and estimate the thickness of the first layer and the depth of the embedded object in the tissue.

As explained earlier, the machine learning model (MLM) includes the first neural network (NN1) and the interaction evaluator (IE), which could include the second neural network (NN2). The first neural network (NN1) can be inputted with measured values of the deformation/displacement, velocity data, and interaction force during tissue manipulation. The first neural network (NN1) characterizes the stiffness and damping of the tissue during tissue manipulation.

The example assumes that the tissue manipulation task transitions from the top layer to the embedded object, and these respective samples are captured in order of this transition. FIG. 13A shows a snapshot of the deformation data for N samples stored in the first LSTM/RNN component of the first neural network (NN1). Each color-coded area of the chart of FIG. 13A represents a layer of the tissue showing where that layer starts deforming during tissue manipulation. FIGS. 13B and FIG. 13C demonstrate the snapshot of the measured velocity data and the measured interaction force for the N samples in first LSTM/RNN during tissue manipulation, respectively.

FIG. 13D shows the measured interaction force graph versus deformation representing the linear relationship between these two variables for each layer. Here, stiffness may be defined as the slope of force-deformation graph with three different values for the three layers of the tissue used in this example. The first neural network (NN1) can be trained to output stiffness values of K1, K2, and K3 when the force and deformation data are inputted to the first neural network (NN1). A similar approach can be applied to output a damping chart, where the force-velocity data are used to measure the damping constant of tissue. We can also generalize this to have all three inputs and estimate both stiffness and damping at the same time.

In addition, the tissue classification and geometrical feature estimation can be achieved by the interaction evaluator (IE), or second neural network (NN2), as described above. Namely, the measured stiffness and damping from the first neural network (NN1) are inputted to the second neural network (NN2) along with the measured velocity. The second neural network (NN2) classifies different types of tissue based on the instantaneous measured stiffness and damping, and the second LSTM2/RNN2 component in the network is responsible for memorizing the change in the stiffness and damping thereby estimating some features of the tissue, for instance the thickness of the first layer and the depth of the embedded object in the tissue.

FIG. 13E shows the stiffness output of the first neural network (NN1) for the given example. As seen in FIG. 13E, the first neural network (NN1) can be used to determine the instantaneous measured stiffness and damping during tissue manipulation. To extract geometrical feature characterization, the second LSTM2/RNN2 component of the second neural network (NN2) can be utilized to memorize the measured stiffness and damping over a pre-defined window (N samples). Analysis of these samples can be used to identify where the transition occurs from one layer to another layer (t1 → t2 and t2 → t3). If the velocity is constant, then the thickness of the respective layer can be measured as: Thickness = Δt x V, where Δt = (t2-t1). However, if the velocity is not constant, the area under the velocity graph (FIG. 13B) between t1 and t2 represents the thickness of the top layer, i.e., denoted by A1 in FIGS. 12 and 13F. For network to compute this, we can discretize the velocity graph and the thickness of the layer can be computed as: Thickness = dt x V(t-N) + dt x V(t-(N-1)) + ... + dt x V(t-(N-N1)), where dt is the sampling time of the control system providing the measurement data. From here, we can see the thickness (A1) of the top layer can be represented as a function of prior velocities between t1 and t2. The depth of the embedded object can be measured as the area under the velocity graph where the transition occurs from the first layer to the third layer, i.e., A1+A2. Here, A2 denotes the thickness of the tissue region from the bottom of the upper layer to the top of the embedded object. Identification of thickness A2 begins when the stiffness K2 is first observed by the machine learning model (MLM) during the penetration task. As the tool 20 continues to penetrate the tissue region, the tool 20 will pass through the second layer (K2 stiffness) and eventually press into the embedded object (stiffness K3). The thickness A3 is the penetration depth of the tool 20 relative to the embedded object defined from a top of the embedded object to the distal tip of the tool 20. Identification of thickness A3 begins when the stiffness K3 is first observed by the machine learning model (MLM) during the penetration task. The thickness A1 + A2 + A3 denotes the total penetration depth of the tool relative to the entire tissue region (i.e., from the top surface of the upper layer of tissue to the distal tip of the tool 20. Using the discretized velocity, the depth can be measured as: Depth = dt x V(t-N) + ... + dt x V(t-(N-N1)) + ... + dt x V(t-(N-N2)). The feature estimation is a function of N velocity inputs (some with the weight of 1 and the rest with the weight of 0, depending on where the transition occurs) that are stored in the LSTM2/RNN2 component of the second neural network (NN2) as follows: Feature = f(V(t-N), ..., V(t)). The second neural network (NN2) represents the (f) function that can be trained to properly estimate the feature.

### E. Use Cases and Examples

Based on any one or more of the above characterizations, the manipulator 14 can be controlled in numerous manners and to perform tasks (as shown in FIG. 7), such as anatomical registration, modification of haptic modification or control, implementing protective actions, implementing adaptive control, and the like. These examples will be described below. However, these examples are not exhaustive of the possible manners in which the described techniques can be utilized. Other solutions are contemplated.

### 1. Anatomical Registration

The predicted characterization(s) can be utilized to register an anatomical region (such as a bone). Such registration can be performed using an imageless technique that does not rely on pre-operative imaging or predetermined bone models. To perform registration, a tool tip touches/traces various surface points of the anatomy. The traces/points can be used to form a point cloud or virtual (3D) surface model of the anatomy. Registration can be performed whether or not the anatomy is tracked by the navigation system 32 using trackers. The tool tip can be the TCP of the tool 20 supported by the manipulator 14. In another example, as described above, the tool tip can be of the pointer P coupled to the adjustable arm (AA) and equipped with the force sensor (S'), and optionally, pointer tracker (PT), e.g., as shown in FIG. 1. Whether the robotically-supported tool 20 or arm-supported pointer P is used, the navigation system 32 can be utilized to record positions of the tool tip over time. Tool tip positions can additionally or alternatively be captured by encoder/joint data from the manipulator 14 or adjustable arm (AA).

The movement of the tool tip into contact with the bone surface can be done automatically (automated mode) or manually in response to user exerted forces (e.g., adjusting the manipulator 14 or adjustable arm AA). As the tool contacts the bone surface, the tool tip positions are recorded by the robotic data and/or navigation data. The bone may optionally be tracked during this process.

Pursuant to the techniques described, the interaction forces, displacements/deformations, and velocities are measured during the bone contact interaction. Based on these measurements, the machine learning model (MLM) estimates the stiffness and damping parameters and characterizes the tissue as bone for the various points collected. The control system 60 then associates the recorded positions of the tool tip to the bone tissue characterizations for the various points to generate the virtual surface model of the bone. The virtual surface model of the bone is then registered to the bone.

The registration technique described is particularly advantageous for complex environments on or around the bone. For example, the machine learning model (MLM) can characterize some points as soft tissue, other points as cartilage, and the majority of points as bone. The recorded positions of the tool tip are then related to the various characterizations for the various points to generate the virtual surface model of the bone, along with an identified soft tissue region and a cartilage map. The soft tissue and cartilage (based on their characterizations) can then be disregarded in the registration process to ensure that the registration accurately reflects only the bone surface. Alternatively, the characterized cartilage and soft tissue regions can be saved and used for other purposes, e.g., by the clinical application 80. In another example, osteophytes can be characterized by the machine learning model (MLM) and localized during the registration process. The characterized osteophyte locations can be used to form an osteophytic virtual surface model or can be disregarded to form a virtual surface model without osteophytes.

### 2. Haptic Modification/Control

In another example, the techniques described above can be utilized to modify the haptics of the manipulator 14 or modify haptic control the manipulator 14. Such modifications can include adjusting the haptic object (Hobj), adjusting parameters of the haptic models (HMa, HMb) for the boundary constraints or active constraints, changing reactive force calculations, or the like.

Modification of the haptics can be responsive to any one or more of the characterizations described. For example, the machine learning model (MLM) may characterize a first portion of the anatomy as comprising a first density/stiffness level and a second portion of the anatomy comprising a second density/stiffness level being denser/stiffer than the first level. These regions may be localized by robotic and/or navigation data, as described above. Thereafter, the control system 60 may define a custom haptic object (Hobj) for each region and/or the boundary constraints can be tuned differently in the boundary constraint model (HMb) for each respective region. In some cases, the haptic object (Hobj) can be temporarily extended based on specific interaction characterizations.

For a spinal procedure, the machine learning model (MLM) may characterize an outer cortical bone layer and a cancellous bone region within a pedicle region. The regions can be characterized by their respective densities and geometries. Based on these characterizations, the active constraint model (HMa) can be tuned to provide stiffer constraints on specified degrees of freedom of movement of the surgical tool 20 in the cancellous region as compared to the outer cortical region.

### 3. Protective Actions

Any of the described characterizations can be used to enable protection from interacting with sensitive, critical, and/or adverse regions. Such regions may be part of the anatomy or separate from the anatomy. Such protections can be afforded using the haptic control techniques described above, or other methods.

For instance, the machine learning model (MLM) can characterize a first tissue type and a second tissue type. The first and second tissue type regions can be localized using robotic data and/or navigation data. In response to this characterization, the control system 60 can generate one or more virtual boundaries VB to delineate the first tissue type region from the second tissue type region. The virtual boundary VB is then registered to the anatomy. The virtual boundary VB is utilized to constrain, limit or prohibit movement or operation of the manipulator 14 and/or the surgical tool 20 relative to the virtual boundary VB. For instance, the first tissue type region can be bone, and the second tissue type region can be soft tissue. The virtual boundary VB enables manipulation of the bone while limiting interaction with the soft tissue.

Similarly, in the example above, the first tissue type region can be healthy tissue, and the second tissue type region can be malignant tissue. Using embedded object and geometrical feature characterization, for example, the machine learning model (MLM) can characterize the size of a tumor and depth of the tumor embedded within soft tissue. In response, the control system 60 can define the virtual boundary VB around the malignant tissue by predefined margins to facilitate removal of the malignant tissue while minimizing risk of recurrence. Similar techniques can be used to define a virtual boundary VB that delineates an anatomical region of interest from any object (e.g., retractor) that may interfere with the region of interest.

### 4. Adaptive Controls

In another example, the control system 60 can adaptively control the manipulator 14 and/or the surgical tool 20 based on any of the described characterizations. Such adaptive control can include any one more of the following: generating or modifying a tool path TP of the surgical tool 20; modifying a feed rate (path velocity) of the surgical tool 20; modifying a cutting speed (e.g., sagittal or rotational) of the surgical tool 20; defining a cutting depth of the surgical tool 20; defining tool path TP oscillation frequencies; adjusting a commanded pose of the surgical tool 20; halting movement of the surgical tool 20; moving the surgical tool 20 towards or away from the anatomy; generating an error; and/or generating or modifying a virtual haptic setting for the surgical tool 20, as described above.

For example, the machine learning model (MLM) can materially characterize a first density level for a first region of a bone, and a second (higher) density level for a second region of the bone. The control system 60 can then utilize the first and second density levels to generate a customized tool path TP that is designed to sculpt both regions in a manner that can improve tool performance or reduce overloading the tool 20. For instance, based on the respective densities, a first part of the tool TP may be defined to remove the first region of bone. The first part of the tool path TP may specify first cutting depth, cutting speed, feed rate, and/or tool path oscillation frequency. A second part of the tool TP (to remove the second portion) may specify a deeper cutting depth, slower cutting speed, slower feed rate, and/or higher tool path oscillation frequency.

In another example, the machine learning model (MLM) can characterize a contact point as an osteophyte. Meanwhile, a virtual bone model may identify the contact point as cortical bone. The control system 60 can compare the contact point information to identify a discrepancy. The control system 60 can then generate an error or feedback. For example, a notification can be presented on the display 38 to prompt the surgeon to remove the osteophyte, and/or the control system 60 can halt operation of the surgical tool 20 and/or manipulator 14 until the error is addressed.

Several embodiments have been described in the foregoing description. However, the embodiments discussed herein are not intended to be exhaustive or limit the invention to any particular form. The terminology, which has been utilized, is intended to be in the nature of words of description rather than of limitation. Many modifications and variations are possible in light of the above teachings and the invention may be practiced otherwise than as specifically described.

## Claims

1. A surgical system (10) comprising:
a robotic manipulator (14) comprising a plurality of links (18) and joints (J);
a surgical tool (20) coupled to the robotic manipulator (14) and being configured to manipulate a tissue of a patient;
a sensing system (60, 26, 74, 76, S, S', 32, AA) configured to measure: displacements of the surgical tool (20), velocities of the surgical tool (20), and interaction forces applied to the surgical tool (20); and
a control system (60, 26, 74, 76) coupled to the robotic manipulator (14) and the sensing system (60, 26, 74, 76, S, S', 32, AA) and being configured to:
control the robotic manipulator (14) to move the surgical tool (20) to interact with the tissue;
responsive to interactions of the surgical tool (20) with the tissue over time, obtain, from the sensing system (60, 26, 74, 76, S, S', 32, AA), measured values of the displacements, velocities, and interaction forces; and
implement a machine learning model (MLM) that is configured to:
receive and process the measured values to estimate stiffness and damping parameters of the tissue; and
monitor changes in the estimated stiffness and damping parameters to predict a tissue characterization; and
wherein the control system (60, 26, 74, 76) is configured to control the robotic manipulator (14) and/or the surgical tool (20) based on the tissue characterization.

2. The surgical system (10) of claim 1, wherein the machine learning model (MLM) comprises a first neural network (NN1) comprising an input layer, hidden layers, and an output layer, wherein:
the input layer of the first neural network (NN1) is configured to receive the measured values; and
the output layer of the first neural network (NN1) is configured to output the estimated stiffness and damping parameters;
and optionally,
wherein the estimated stiffness and damping parameters are embeddings of lower dimensionality than the measured values.

3. The surgical system (10) of claim 2, wherein the machine learning model (MLM) comprises a first long short-term memory (LSTM) coupled to the input layer of the first neural network (NN1), wherein the first LSTM comprises a recurrent neural network (RNN) and is configured to:
receive the measured values over time;
modify weights of the RNN to learn long-term dependencies among the measured values and to selectively filter the measured values; and
provide filtered measured values to the input layer of the first neural network (NN1).

4. The surgical system (10) of any one of claims 2 to 3, wherein the machine learning model (MLM) comprises a second neural network (NN2) comprising an input layer, hidden layers, and an output layer, wherein:
the input layer of the second neural network (NN2) is configured to receive the estimated stiffness and damping parameters; and
the output layer of the second neural network (NN2) is configured to output the tissue characterization, wherein the tissue characterization is represented by a vector of higher dimensionality than the estimated stiffness and damping parameters;
and optionally,
wherein the machine learning model (MLM) comprises a second LSTM coupled to the input layer of the second neural network (NN2), wherein the second LSTM comprises a second RNN and is configured to:
receive, over time, the estimated stiffness and damping parameters from the output layer of the first neural network (NN1);
modify weights of the second RNN to learn long-term dependencies among the estimated stiffness and damping parameters and to selectively filter the estimated stiffness and damping parameters; and
provide filtered estimated stiffness and damping parameters to the input layer of the second neural network (NN2).

5. The surgical system (10) of any preceding claim, wherein the tissue characterization comprises a parameter of the tissue, wherein the parameter of the tissue includes one or more of: geometry, size, shape, depth, thickness, density, stiffness, hardness, softness, smoothness, and roughness.

6. The surgical system (10) of any preceding claim, wherein the tissue characterization comprises one or more of: a layering of the tissue, and identification of the tissue being an embedded object.

7. The surgical system (10) of any preceding claim, wherein the tissue characterization comprises a tissue type, and optionally, wherein the tissue type comprises one or more of: bone, an osteophyte, cartilage, soft tissue, muscle, ligament tissue, tendon tissue, a blood vessel, healthy tissue, and malignant tissue.

8. The surgical system (10) of claim 7, wherein:
the machine learning model (MLM) is configured to monitor changes in the estimated stiffness and damping parameters to predict a first tissue type and to predict a second tissue type; and
the control system (60, 26, 74, 76) is configured to:
record positions of the surgical tool (20) responsive to interactions of the surgical tool (20) with the first tissue type and the second tissue type over time; and
utilize the recorded positions to register the first tissue type and the second tissue type to locations on the tissue.

9. The surgical system (10) of claim 8, wherein the control system (60, 26, 74, 76) is further configured to:
generate a virtual boundary (VB) to delineate a first region of the tissue comprising the first tissue type from a second region of the tissue comprising the second tissue type;
register the virtual boundary (VB) to the tissue; and
utilize the virtual boundary (VB) to constrain movement or operation of the robotic manipulator (14) and/or the surgical tool (20) relative to the virtual boundary (VB).

10. The surgical system (10) of any one of claims 7 to 9, wherein:
the machine learning model (MLM) is configured to monitor changes in the estimated stiffness and damping parameters to predict that the tissue type is a bone; and
the control system (60, 26, 74, 76) is configured to:
record positions of the surgical tool (20) responsive to interactions of the surgical tool (20) with the bone over time; and
utilize the recorded positions to generate a 3-D surface model of the bone and register the 3-D surface model to the bone.

11. The surgical system (10) of any preceding claim, wherein the control system (60, 26, 74, 76) controls the robotic manipulator (14) and/or the surgical tool (20) based on the tissue characterization by being configured to perform one or more of the following:
modify a tool path (TP) of the surgical tool (20);
modify a feed rate of the surgical tool (20);
modify a cutting speed of the surgical tool (20);
adjust a commanded pose of the surgical tool (20);
halt movement of the surgical tool (20);
move the surgical tool (20) towards or away from the tissue; and/or
generate or modify a virtual haptic setting for the surgical tool (20).

12. The surgical system (10) of any preceding claim, wherein:
the control system (60, 26, 74, 76) is configured to compute a deformation of the tissue based on the measured values of displacements and interaction forces; and
the machine learning model (MLM) is configured to further receive and process values of the deformation to estimate stiffness and damping parameters of the tissue.

13. The surgical system (10) of any preceding claim, wherein the control system (60, 26, 74, 76) is configured to:
record positions of the surgical tool (20) responsive to interactions of the surgical tool (20) with the tissue over time; and
utilize the recorded positions to register the tissue characterization to locations on the tissue.

14. A method of operating the surgical system (10) of any preceding claim.

15. A non-transitory computer readable medium, or computer program product, comprising instructions, which when executed by one or more processors, are configured to implement the surgical system (10) of any one of claims 1 to 13.
